# EUROPEAN PATENT APPLICATION

(11) **EP 4 339 285 A1**
(43) Date of publication of application: **20.03.2024**
(21) Application number: 22807791.3
(22) Date of filing: 10.05.2022
(51) Int. Cl.: C12N 15/113, C12Q 1/6886

(54) **GUIDE RNA FOR DETECTING ONCOGENIC MUTANT GENE AND USE THEREOF**

(30) Priority: 11.05.2021 KR 20210061037
(71) Applicant: Aima Co., Ltd., Seoul 03722 (KR)
(72) Inventor: KIM, Hyongbum, Seoul 04091 (KR); KIM, Sangwoo, Seoul 04104 (KR); KIM, Hyeryun, Seoul 03709 (KR); SON, Hyeonju, Seoul 03721 (KR); GOPALAPPA, Ramu, Seoul 03787 (KR)
(74) Representative: Holt, Lucy Rose
(86) International application number: PCT/KR2022/006665
(87) International publication number: WO 2022/240143

(57) **Abstract**

The present invention relates to a guide RNA used for detecting an oncogenic mutant gene by cleaving a wild-type cancer cell gene by using CRISPR/Cas9, and to a use thereof.

When cancer mutation is detected using a CRISPR/Cas9 system by using the guide RNA of the present invention, only wild-type cell-free DNA (cfDNA) is cut and oncogenic mutant DNA is not cut. Thus, as compared to when a guide RNA of an existing CRISPR/Cas9 system is used, the guide RNA of the present invention can be effectively used to selectively amplify only oncogenic mutant DNA.

## Description

### [Technical Field]

This application claims priority to and the benefit of Korean Patent Application No. 10-2021-0061037, filed on May 11, 2021, the disclosure of which is incorporated herein by reference in its entirety.

The present invention relates to a guide RNA that is used for detecting an oncogenic mutant gene by cleaving a wild-type cancer cell gene using the CRISPR/Cas9 system and a use thereof.

This application was supported by the Ministry of Health and Welfare (No. HR14C0005020021 (1465033586)).

### [Background Art]

Based on advances in sequencing technology, it is known that cancer is usually caused by oncogenic mutations. Several studies have provided evidence that tumorigenesis is closely related to the prevalence of somatic mutations in certain types of cancer. The COSMIC (Catalogue of Somatic Mutations in Cancer) database includes hundreds of thousands of human cancer-associated somatic mutations, which are classified by tumor type and disease.

Along with this, liquid biopsy has recently emerged as a useful alternative to conventional tissue biopsy, providing a non-invasive approach to detect and monitor tumor-derived elements circulating in the bloodstream, and it can be used directly or indirectly as a source of cancer biomarkers. Blood contains two types of cancer-derived components that are susceptible to detailed molecular analysis. One is intact circulating tumor cells (CTCs) and cell-free circulating tumor DNA (cfDNA or circulating tumor DNA; ctDNA). As tumor volume increases, the ability of phagocytes to clear and remove apoptotic and necrotic fragments may be exceeded, resulting in the passive release of cfDNA into the bloodstream. This liquid biopsy helps analyze the DNA that the tumor normally releases into the bloodstream. Depending on tumor size, the amount of cfDNA released into the bloodstream varies from 0.01% to 90% of all DNA present in plasma. Therefore, liquid biopsy provides a large-scale, non-invasive approach to tumor molecular profiling without obtaining tumor tissue.

However, cfDNA in plasma usually contains very small amounts of tumor DNA, which is known to vary with tumor burden or cancer stage. Therefore, a highly sensitive and specific method is required to diagnose tumors by detecting ctDNA, especially in the early stages of cancer. CRISPR (Clustered regular interspaced short palindromic repeats) and Cas (CRISPR-associated) protein systems, which are the adaptive immune responses of prokaryotic organisms, are well known for specific DNA target recognition and cleavage. The versatility of the CRISPR system has been extensively utilized by us and other groups for genome editing in a variety of organisms as well as cleaving DNA *in vitro.* CRISPR endonucleases (e.g., type II Cas9 or type V Cpf1) respectively recognize a protospacer-adjacent motif (PAM) downstream or upstream of the target DNA sequence corresponding to the guide RNA (gRNA), thereby inducing double-strand breakage in the target DNA. The use of ctDNA for cancer diagnosis requires the detection of a small amount of ctDNA with a missense mutation in a highly accurate manner among a relatively large amount of wild-type cfDNA. Tumor-specific versus wild-type alleles can be enriched by specifically cleaving the wild-type DNA, and in order to cleave the wild-type target DNA using Cas9-sgRNA, a single guide RNA must be designed to target the sequence of the PAM site. However, since the DNA PAM site targeted in cfDNA is destroyed by oncogenic mutation, there exists a problem in that it is difficult to detect because it is not recognized by CRISPR endonuclease.

### [Disclosure]

### [Technical Problem]

Accordingly, the inventors of the present invention have made diligent efforts to provide a system for detecting cancer cells by using CRISPR endonuclease and Cas9 (CRISPR/Cas9) for cancer diagnosis, which can also be utilized for cancer-related mutations in which PAM sequences do not exist, and as a result, the present invention was completed by fabricating a guide RNA which is capable of specifically cleaving only wild-type DNA, not mutant DNA of cancer cells.

Accordingly, an object of the present invention is to provide a guide RNA of the CRISPR/Cas9 system or a composition or kit for diagnosing cancer or predicting cancer prognosis including the same.

Another object of the present invention is to provide a method for providing information for diagnosing cancer or predicting cancer prognosis, including the step of treating a guide RNA of the CRISPR/Cas9 system to a sample of a patient.

### [Technical Solution]

The present invention provides a guide RNA, which is a guide RNA used in the CRISPR/Cas9 system, wherein the guide RNA cleaves wild-type cell-free DNA.

According to a preferred exemplary embodiment of the present invention, the guide RNA may include at least any one nucleotide sequence selected from the group consisting of SEQ ID NOs: 1 to 17.

According to a preferred exemplary embodiment of the present invention, in the guide RNA, a transversion mutation may have occurred in at least any one region selected from the group consisting of the trunk region, semi-seed region and seed region of the guide RNA.

According to a preferred exemplary embodiment of the present invention, the guide RNA may include at least any one nucleotide sequence selected from the group consisting of SEQ ID NO: 2, SEQ ID NO: 4, SEQ ID NO: 9 and SEQ ID NO: 13.

According to a preferred exemplary embodiment of the present invention, the guide RNA may be capable of detecting at least any one cancer-related mutation selected from the group consisting of EGFR, MET, KRAS, PIK3CA and BRAF.

According to a preferred exemplary embodiment of the present invention, when detecting the EGFR mutation, the guide RNA may include at least any one nucleotide sequence selected from the group consisting of SEQ ID NOs: 1 to 4, 8, 9 and 11 to 13.

According to a preferred exemplary embodiment of the present invention, when detecting the MET mutation, the guide RNA may include at least any one nucleotide sequence selected from the group consisting of SEQ ID NOs: 5 to 7.

According to a preferred exemplary embodiment of the present invention, when detecting the KRAS mutation, the guide RNA may include the nucleotide sequence of SEQ ID NO: 10.

According to a preferred exemplary embodiment of the present invention, when detecting the PIK3CA mutation, the guide RNA may include at least any one nucleotide sequence selected from the group consisting of SEQ ID NOs: 14 to 16.

According to a preferred exemplary embodiment of the present invention, when detecting the BRAF mutation, the guide RNA may include the nucleotide sequence of SEQ ID NO: 17.

The present invention provides a composition for diagnosing cancer or predicting cancer prognosis, including a guide RNA of CRISPR/Cas9 including at least any one nucleotide sequence selected from the group consisting of SEQ ID NOs: 1 to 17.

According to a preferred exemplary embodiment of the present invention, in the guide RNA, a transversion mutation may have occurred in at least any one region selected from the group consisting of the trunk region, semi-seed region and seed region of the guide RNA.

According to a preferred exemplary embodiment of the present invention, the guide RNA may be capable of detecting at least any one cancer-related mutation selected from the group consisting of EGFR, MET, KRAS, PIK3CA and BRAF.

According to a preferred exemplary embodiment of the present invention, the guide RNA may cleave wild-type cell-free DNA.

According to a preferred exemplary embodiment of the present invention, the cancer may be at least any one cancer selected from the group consisting of lung cancer, pancreatic cancer, colon cancer, stomach cancer, breast cancer, kidney cancer, liver cancer, blood cancer, bladder cancer and uterine cancer.

In addition, the present invention provides a kit for diagnosing cancer or predicting cancer prognosis, including the composition.

In addition, the present invention provides a method for providing information for diagnosing cancer or predicting cancer prognosis, including the step of treating the composition to a sample of a patient.

As described above, the detection of oncogenic mutant genes using cell-free DNA with the existing CRISPR/Cas9 system has had limitations in detecting oncogenic mutant genes, such as low detection probability with guide RNA, by targeting mutations in three PAM sequence (NGG) nucleotides.

In order to overcome the above limitations, the guide RNA of the present invention targets and cleave wild-type cell-free DNA, not oncogenic mutant genes, through the CRISPR/Cas9 system, and particularly, by using a guide RNA that has undergone a transversion mutation in the trunk region of the guide RNA, only oncogenic mutant genes can be selectively targeted and amplified.

Accordingly, the present invention is a guide RNA used in the CRISPR/Cas9 system, wherein the guide RNA may cleave wild-type cell-free DNA.

The 'CRISPR/Cas9 system' refers to a system in which an enzyme called Cas9, which can cut genes by recognizing a specific base sequence, can cut specific DNA by recognizing a single-stranded guide RNA (sgRNA) including the nucleotide sequence thereof. The CRISPR/Cas9 system consists of sgRNA, which is designed to accurately recognize target DNA, and Cas9, which can cleave target DNA based thereon, and Cas9, which recognizes sgRNA, cuts the DNA double helix.

According to a preferred exemplary embodiment of the present invention, the guide RNA may include at least any one nucleotide sequence selected from the group consisting of SEQ ID NOs: 1 to 17.

The guide RNA including the nucleotide sequence of SEQ ID NO: 1 is a wild-type guide RNA (wild sgRNA) which is capable of detecting EGFR (p.T790M) mutation, and when it is used in an *in vitro* cleavage analysis, it may have SEQ ID NO: 18 as a target sequence.

The guide RNA including the nucleotide sequence of SEQ ID NO: 2 is a transversion guide RNA (transversion sgRNA) which is capable of detecting EGFR (p.T790M) mutation, and when it is used for an *in vitro* cleavage analysis, it may have SEQ ID NO: 19 as a target sequence. When using a guide RNA including the nucleotide sequence of SEQ ID NO: 2, the mutant sequence may be amplified by 1% to 32%.

The guide RNA including the nucleotide sequence of SEQ ID NO: 3 is a wild-type guide RNA (wild sgRNA) which is capable of detecting EGFR (p.C797S) mutation, and when it is used in an *in vitro* cleavage analysis, it may have SEQ ID NO: 20 as a target sequence.

The guide RNA including the nucleotide sequence of SEQ ID NO: 4 is a transversion guide RNA (transversion sgRNA) which is capable of detecting EGFR (p.C797S) mutation, and when it is used in an *in vitro* cleavage analysis, it may have SEQ ID NO: 21 as a target sequence. When using the guide RNA including the nucleotide sequence of SEQ ID NO: 4, the mutant sequence can be amplified by 1% to 13%.

The guide RNA including the nucleotide sequence of SEQ ID NO: 5 is a wild-type guide RNA (wild sgRNA) which is capable of detecting MET c.2888 Int13-Exon14 Acceptor (Exon-14 Skipping) mutation, and when it is used in an *in vitro* cleavage analysis, it may have SEQ ID NO: 22 or 23 as a target sequence.

The guide RNA including the nucleotide sequence of SEQ ID NO: 6 is a wild-type guide RNA (wild sgRNA) which is capable of detecting MET c.3028 Exon14-Int14 Donor (Exon-14 Skipping) mutation, and when it is used in an *in vitro* cleavage analysis, it may have SEQ ID NO: 24 or 25 as a target sequence.

The guide RNA including the nucleotide sequence of SEQ ID NO: 7 is a wild-type guide RNA (wild sgRNA) which is capable of detecting MET 'TAC' Y1003X (Exon-14 Skipping) mutation, and when it is used in an *in vitro* cleavage analysis, it may have SEQ ID NO: 26 or 27 as a target sequence.

The guide RNA including the nucleotide sequence of SEQ ID NO: 8 is a wild-type guide RNA (wild sgRNA) which is capable of detecting EGFR (L718Q) mutation, and when it is used in an *in vitro* cleavage analysis, it may have SEQ ID NO: 28 as a target sequence.

The guide RNA including the nucleotide sequence of SEQ ID NO: 9 is a transversion guide RNA (transversion sgRNA) which is capable of detecting EGFR (L718Q) mutation, and when it is used in an *in vitro* cleavage analysis, it may have SEQ ID NO: 29 as a target sequence. When using the guide RNA including the nucleotide sequence of SEQ ID NO: 9, the mutant sequence may be amplified by 0.8% to 23.7%.

The guide RNA including the nucleotide sequence of SEQ ID NO: 10 is a wild-type guide RNA (wild sgRNA) which is capable of detecting KRAS (c.35G) mutation, and when it is used in an *in vitro* cleavage analysis, it may have SEQ ID NO: 30 or 31 as a target sequence. When using the guide RNA including the nucleotide sequence of SEQ ID NO: 10, the mutant sequence may be amplified by 1.5% to 50%.

The guide RNA including the nucleotide sequence of SEQ ID NO: 11 is a wild-type guide RNA (wild sgRNA) which is capable of detecting EGFR, 15 bp Del p. (729_761) mutation, and when it is used in an *in vitro* cleavage analysis, it may have SEQ ID NO: 32 or 33 as a target sequence. When using the guide RNA including the nucleotide sequence of SEQ ID NO: 11, the mutant sequence may be amplified by 0.5% to 23%.

The guide RNA including the nucleotide sequence of SEQ ID NO: 12 is a wild-type guide RNA (wild sgRNA) which is capable of detecting EGFR (p.L858R) mutation, and when it is used in an *in vitro* cleavage analysis, it may have SEQ ID NO: 34 as a target sequence.

The guide RNA including the nucleotide sequence of SEQ ID NO: 13 is a transversion guide RNA (transversion sgRNA) which is capable of detecting EGFR (p.L858R) mutation, and when it is used in an *in vitro* cleavage analysis, it may have SEQ ID NO: 35 as a target sequence. When using the guide RNA including the nucleotide sequence of SEQ ID NO: 13, the mutant sequence may be amplified by 1.7% to 63%.

The guide RNA including the nucleotide sequence of SEQ ID NO: 14 is a wild-type guide RNA (wild sgRNA) which is capable of detecting PIK3CA (p.E542K or c.1624G>A) mutation, and when it is used in an *in vitro* cleavage analysis, it may have SEQ ID NO: 36 or 37 as a target sequence.

The guide RNA including the nucleotide sequence of SEQ ID NO: 15 is a wild-type guide RNA (wild sgRNA) which is capable of detecting PIK3CA (p.E545? or c.1633G>?) mutation, and when it is used in an *in vitro* cleavage analysis, it may have SEQ ID NO: 38 or 39 as a target sequence.

The guide RNA including the nucleotide sequence of SEQ ID NO: 16 is a wild-type guide RNA (wild sgRNA) which is capable of detecting PIK3CA (p.H1047? or c.3140A>?) mutation, and when it is used in an *in vitro* cleavage analysis, it may have SEQ ID NO: 40 or 41 as a target sequence.

The guide RNA including the nucleotide sequence of SEQ ID NO: 17 is a wild-type guide RNA (wild sgRNA) which is capable of detecting PIK3CA (p.V600E or c.1799T>A) mutation, and when it is used in an *in vitro* cleavage analysis, it may have SEQ ID NO: 42 or 43 as a target sequence.

The guide RNA may be a guide RNA, in which a transversion mutation has occurred in at least any one region selected from the group consisting of the trunk region, semi-seed region and seed region of the guide RNA.

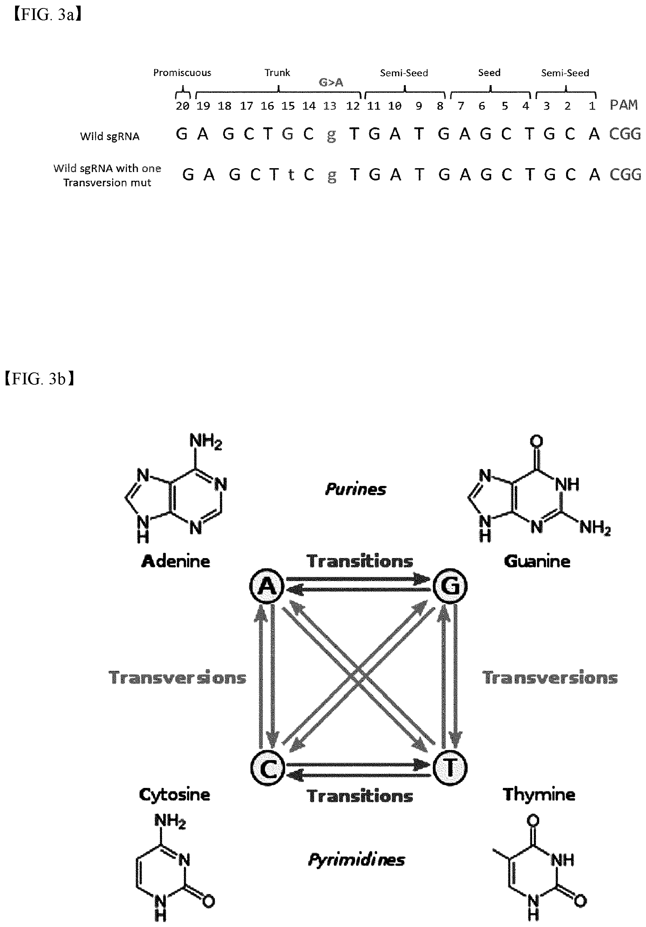

Targeting DNA cleavage by SpCas9 mainly requires specific 20 nucleotides of the target protospacer sequences along with 3 PAM sequence (NGG) nucleotides that are adjacent to the 3' end of the target sequence. Mutations within the PAM sequence (caused by either mismatched PAM-NGN or any oncogenic mutation) are the best candidates that are easy to enrich the mutant allele in wild-type DNA by causing Cas9 to cleave from cell-free DNA to the wild-type target allele (PAM-NGG). However, it is not possible to have such discordant PAM target sequences for all cancer-associated mutations. The inventors of the present invention have prepared a transversion mutation (purine to pyrimidine) in the trunk region of a guide RNA to solve the above problems and target all types of mutations. Depending on the resistance to sgRNA activity, single-base mismatches in a guide RNA are classified into semi-seed, seed, trunk and promiscuous regions. The guide RNA resistance to a single-base mismatch within the seed region from position 4 to 7 is the lowest resistance of the corresponding sgRNA. Next, both sides of the seed region at positions 1-3 and 8-11 are medium tolerance for mismatches and are defined as semi-seed regions. A single-base mismatch at positions 12-19 reduces the cleavage frequency to an intermediate level but retained sgRNA activity, and this intermediate region is termed the trunk region. At position 20, a single-base mismatch slightly reduces the frequency of cleavage induced by the Cas9 nuclease, and this position is termed a promiscuous region. Based on this strategy, the Cas9 nuclease can be treated with a transversion mutation guide RNA to enrich for the low-frequency mutant allele, followed by PCR amplification to remove the wild-type target allele. Mutant target alleles are not recognized or cleaved by the Cas9 nuclease. This is because of the low resistance to two mutations, that is, the first is a conversion mutation in the sgRNA, and the second is a target mutation of the oncogenic mutation (FIG. 3a, FIG. 3b).

The Cas9 nuclease may include various variants and may be Cas9, eCas9 or HFCas9.

According to a preferred exemplary embodiment of the present invention, the guide RNA in which the transversion mutation has occurred may include at least any one nucleotide sequence selected from the group consisting of SEQ ID NO: 2, SEQ ID NO: 4, SEQ ID NO: 9 and SEQ ID NO: 13.

According to a preferred exemplary embodiment of the present invention, the guide RNA may detect at least any one cancer-related mutation selected from the group consisting of EGFR, MET, KRAS, PIK3CA and BRAF.

According to a preferred exemplary embodiment of the present invention, when detecting the EGFR mutation, the guide RNA may include at least any one nucleotide sequence selected from the group consisting of SEQ ID NOs: 1 to 4, 8, 9 and 11 to 13.

According to a preferred exemplary embodiment of the present invention, when detecting the MET mutation, the guide RNA may include at least any one nucleotide sequence selected from the group consisting of SEQ ID NOs: 5 to 7.

According to a preferred exemplary embodiment of the present invention, when detecting the KRAS mutation, the guide RNA may include the nucleotide sequence of SEQ ID NO: 10.

According to a preferred exemplary embodiment of the present invention, when detecting the PIK3CA mutation, the guide RNA may include at least any one nucleotide sequence selected from the group consisting of SEQ ID NOs: 14 to 16.

According to a preferred exemplary embodiment of the present invention, when detecting the BRAF mutation, the guide RNA may include the nucleotide sequence of SEQ ID NO: 17.

In addition, the present invention may provide a guide RNA of CRISPR/Cas9, including at least any one nucleotide sequence selected from the group consisting of SEQ ID NOs: 1 to 17. The guide RNA may cleave wild-type cell-free DNA.

According to a preferred exemplary embodiment of the present invention, in the guide RNA, a transversion mutation may have occurred in at least any one region selected from the group consisting of the trunk region, semi-seed region and seed region of the guide RNA.

In addition, the present invention may provide a composition for diagnosing cancer or predicting cancer prognosis, including the guide RNA.

According to a preferred exemplary embodiment of the present invention, the cancer may be at least any one cancer selected from the group consisting of lung cancer, pancreatic cancer, colon cancer, stomach cancer, breast cancer, kidney cancer, liver cancer, blood cancer, bladder cancer and uterine cancer, but any cancer caused by EGFR, MET, KRAS, PIK3CA or BRAF mutation may be eligible without limitation.

In addition, the present invention may provide a kit for diagnosing cancer or predicting cancer prognosis, including the composition.

The kit may consist of one or more other component compositions, solutions or devices that are suitable for commonly used expression level analysis methods. For example, a kit for measuring protein expression levels may include a substrate, an appropriate buffer, a secondary antibody labeled with a chromogenic enzyme or a fluorescent substance, a chromogenic substrate and the like for immunological detection of the antibody.

The kit of the present invention may include a sample extraction means for obtaining a sample from an evaluation subject. The sample extraction means may include a needle or a syringe. The kit may include a sample collection container for receiving an extracted sample, which may be liquid, gaseous or semi-solid. The kit may further include instructions for use. The sample may be any body sample in which an oncogenic mutant gene may be present or secreted. For example, the sample may be urine, feces, hair, sweat, saliva, bodily fluid, blood, cells or tissue. The measurement of oncogenic mutant genes in a body sample may be performed on whole samples or processed samples.

In addition, the present invention may provide a method for providing information for diagnosing cancer or predicting cancer prognosis, including the step of treating the guide RNA to a sample of a patient.

The sample may be urine, feces, hair, sweat, saliva, body fluid, blood, cells or tissue.

### [Advantageous Effects]

When detecting cancer mutations with the CRISPR/Cas9 system using the guide RNA of the present invention, only wild-type cell-free DNA (cfDNA) is cleaved and oncogenic mutant DNAis not cleaved, and thus, compared to when the guide RNA of the existing CRISPR/Cas9 system is used, it can be effectively used to selectively amplify only oncogenic mutant DNA. In particular, when the guide RNA (wild-type guide RNA) of the present invention and the transversion guide RNA (transversion sgRNA) are used together, the effects of cleaving only cfDNA and selectively amplifying only oncogenic mutant DNA can be significantly increased.

### [Description of Drawings]

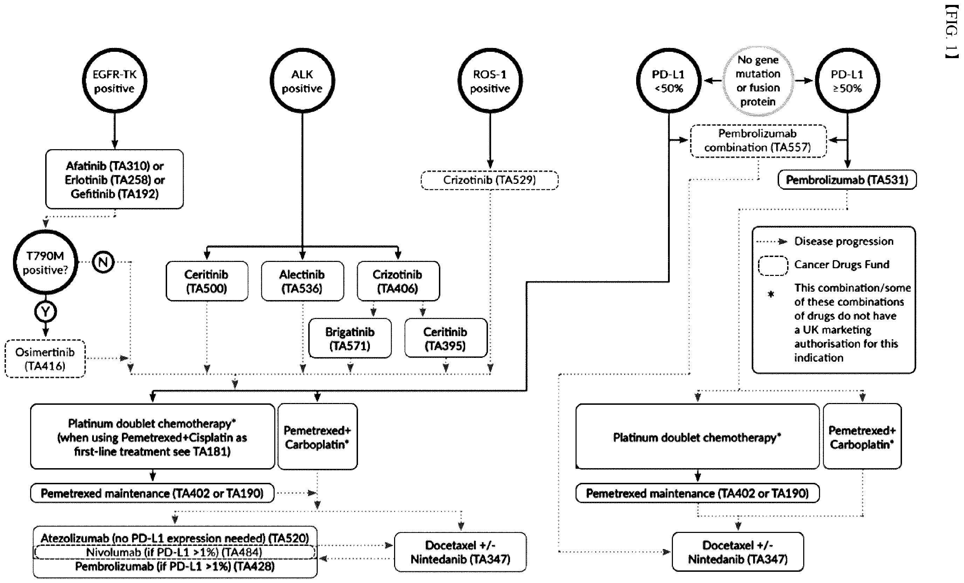

FIG. 1 shows a summary with the NICE guidelines for the disease progression and options for drug therapy or systemic chemotherapy for patients with non-squamous (adenocarcinoma; EGFR-TK, ALK or ROS-1 positive) carcinoma and non-small cell carcinoma for lung cancer.

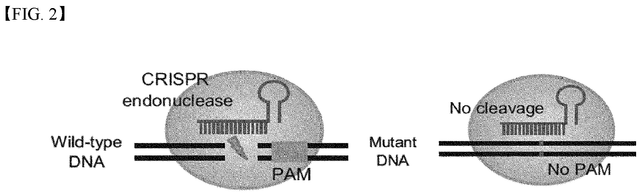

FIG. 2 shows a schematic diagram for amplifying mutant alleles in cell-free DNA (cfDNA) by using Cas9-sgRNA.
FIGS. 3a and 3b show a guide RNA design strategy to amplify all possible mutant alleles with Cas9 treatment in cell-free DNA (cfDNA).
FIGS. 4a and 4b show an *in vitro* cleavage assay for detecting a KRAS mutant (c.35G>C) sequence by using the guide RNA of the present invention and the results thereof.
FIG. 5 shows an *in vitro* cleavage assay for detecting an EGFR mutation (c.2369C>T or p.T790M) sequence by using the guide RNA of the present invention and the results thereof.
FIGS. 6a and 6b show an *in vitro* cleavage assay for detecting an EGFR mutation (c.2573 T>G or p.L858R) sequence by using the guide RNA of the present invention and the results thereof.
FIG. 7 shows an *in vitro* cleavage assay for detecting an EGFR mutation (c.2573 T>G or p.L858R) sequence in a patient (LA-29) sample by using the guide RNA of the present invention and the results thereof.
FIG. 8 shows an *in vitro* cleavage assay for detecting an EGFR mutation (p.C7975, L718Q or p.(729_761) loss) or MET mutation (c.2888, c.3028 or 'TAC'Y1003X) sequence by using the guide RNA of the present invention and the results thereof.
FIG. 9 shows an *in vitro* cleavage assay for detecting a BRAF mutation (p.V600E or c.1799T>A) or PIK3CA mutation (c.1624G>A or p.E542K; p.H1047? or c.3140A>?) sequence by using the guide RNA of the present invention and the results thereof.
FIG. 10 shows the PCR results of detecting an EGFR mutation (T790M) sequence by using the guide RNA of the present invention.
FIG. 11 quantitatively shows the PCR results of detecting an EGFR mutation (T790M) sequence by using the guide RNA of the present invention.
FIG. 12 shows the results of treating Cas9 after mixing a wild-type gene and a mutant gene (KRAS c.35G>C) at various ratios.
FIG. 13 shows the results of treating Cas9 after mixing a wild-type gene and a mutant gene (EGFR-c.2369C>T or p.T790M) at various ratios.
FIG. 14 shows the results of treating Cas9 after mixing a wild-type gene and a mutant gene (EGFR c.2390G>C or p.C797S) at various ratios.
FIG. 15 shows the results of treating Cas9 after mixing a wild-type gene and a mutant gene (EGFR c.2153T to A (L718Q)) at various ratios.
FIG. 16 shows allele frequency and copy number data collected by ddPCR (Droplet digital PCR) as a cfDNA reference standard set. Other endogenous variants were pre-established, but only the data presented were tested within each batch (the variant was detected by ddPCR, although not confirmed for the expected AF due to assay background; since probe competition with NRAS Q61K was demonstrated due to the QC assay used, it was not confirmed for the expected AF, but the variant was detected by ddPCR).
FIG. 17 shows the PCR results for mutant genes using the guide RNA of the present invention based on a cfDNA reference standard set.
FIG. 18 shows a workflow for cell-free DNA detection in patient samples.
FIG. 19 shows the results of detecting the L858R mutation among the EGFR mutations in patient (LA-29, LA-209 or LA-292) samples by using the guide RNA and Cas9 of the present invention.
FIG. 20 shows the results of single detection of the T790M or E19del mutation among the EGFR mutations in patient (LA-232) samples by using the guide RNA and Cas9 of the present invention.
FIG. 21 shows the results of multiple detection of the T790M or E19del mutation among EGFR mutations in patient (LA-232) samples by using the guide RNA and Cas9 of the present invention.
FIG. 22 shows the results of single detection of the T790M or E19del mutation among EGFR mutations in patient (LA-322) samples by using the guide RNA and Cas9 of the present invention.
FIG. 23 shows the results of multiple detection of the T790M or E19del mutation among EGFR mutations in patient (LA-322) samples by using the guide RNA and Cas9 of the present invention.

### [Best Mode]

### [Example 1]

### Fabrication of guide RNAs for drug resistance mutations

Wild-type and mutant target sequences of selected genes and guide RNA sequences were prepared for testing an *in vitro* transcription cleavage (IVT) analysis.

**[Table 1]**

| **Gene/ Mut.AA** | **Nucleotide (WT/Mut)** | **In-vitro Cleavage Assay Target Sequence** | **sgRNA** + **PAM Sequence** | **sgRNA Type** |
|---|---|---|---|---|
| EGFR (p.T790M or c.2369C> T) | WT - C | TCACCTCCACCGTGCAGCTCATCAcGCA GCTCATGCCCTTC (SEQ ID NO: 18) | CTGCgTGATGAGCTG CACGGTGG (SEQ ID NO: 1) | Wild sgRNA |
| | Mut - T | TCACCTCCACCGTGCAGCTCATCAtGCA GCTCATGCCCTTC (SEQ ID NO: 19) | CTGCgTGcTGAGCTG CACGGTGG (SEQ ID NO: 2) | Transver sion sgRNA |
| EGFR (p.C797S or c.2390 G> C) | WT - G | ACGCAGCTCATGCCCTTCGGCTgCCTCC TGGACTATGTCCG (SEQ ID NO: 20) | CATGCCCTTCGGCTg CCTCCTGG (SEQ ID NO: 3) | Wild sgRNA |
| | Mut - C | ACGCAGCTCATGCCCTTCGGCTcCCTCC TGGACTATGTCCG (SEQ ID NO: 21) | CATGCaCTTCGGCTg CCTCCTGG (SEQ ID NO: 4) | Transver sion sgRNA |
| MET c.2888 Int13-Exon14 Acceptor (Exon-14 Skipping) | WT - GA | TTAACAAGCTCTTTCTTTCTCTCTGTTT TAAgaTCTGGGCA (SEQ ID NO: 22) | TTTCTCTCTGTTTTA AgaTCTGG (SEQ ID NO: 5) | Wild sgRNA |
| | Mut - ?? | TTAACAAGCTCTTTCTTTCTCTCTGTTT TAAgaTCTGGGCA (SEQ ID NO: 23) | TTTCTCTCTGTTTTA AgaTCTGG | |
| MET c.3028 Exon14-Int14 Donor (Exon-14 Skipping) | WT - AC | ATTTCTCAGAACAATAAACTGAAATAT acCTTCTGGAAAAG (SEQ ID NO: 24) | ATAAACTGAAATAT acCTTCTGG (SEQ ID NO: 6) | Wild sgRNA |
| | Mut - ?? | ATTTCTCAGAACAATAAACTGAAATAT acCTTCTGGAAAAG (SEQ ID NO: 25) | ATAAACTGAAATAT acCTTCTGG | |
| MET 'TAC' Y1003X (Exon-14 | WT - TAC | GAAATGGTTTCAAATGAATCTGTAGAC tacCGAGCTACTTT (SEQ ID NO: 26) | AAATGAATCTGTAG ACtacCGAG (SEQ ID NO: 7) | Wild sgRNA |
| Skipping) | Mut - ??? | GAAATGGTTTCAAATGAATCTGTAGAC tacCGAGCTACTTT (SEQ ID NO: 27) | AAATGAATCTGTAG ACtacCGAG | |
| EGFR (L718Q or c.2153 T> A) | WT - T | GAATTCAAAAAGATCAAAGTGCtGGGC TCCGGTGCGTTCGG (SEQ ID NO: 28) | AAGATCAAAGTGCt GGGCTCCGG (SEQ ID NO: 8) | Wild sgRNA |
| | Mut - A | GAATTCAAAAAGATCAAAGTGCaGGGC TCCGGTGCGTTCGG (SEQ ID NO: 29) | AAGATCAcAGTGCtG GGCTCCGG (SEQ ID NO: 9) | Transver sion sgRNA |
| KRAS (c.35G) | WT - G | TGACTGAATATAAACTTGTGGTAGTTG GAGCTGgTGGCGTAGGCA (SEQ ID NO: 30) | AAACTTGTGGTAGT TGGAGCTGG (SEQ ID NO: 10) | Wild sgRNA |
| | Mut - C (A or T) | TGACTGAATATAAACTTGTGGTAGTTG GAGCTGcTGGCGTAGGCA (SEQ ID NO: 31) | AAACTTGTGGTAGT TGGAGCTGg | |
| EGFR, 15bp Del p.(729_761) | WT - GGAATTAA GAGAAGC | AAGTTAAAATTCCCGTCGCTATCAAgga attaagagaagcAACATC (SEQ ID NO: 32) | TAAAATTCCCGTCG CTATCAAGG (SEQ ID NO: 11) | Wild sgRNA |
| | Mut - **15bp** Deletion | AAGTTAAAATTCCCGTCGCTATCAAgga attaagagaagcAACATC (SEQ ID NO: 33 | TAAAATTCCCGTCG CTATCAAGG | |
| EGFR (p.L858R or c.2573 T>G) | WT - T | GATCACAGATTTTGGGCtGGCCAAACT GCTGGGTGCGGAAG (SEQ ID NO: 34) | TTTTGGGCtGGCCAA ACTGCTGG (SEQ ID NO: 12) | Wild sgRNA |
| | Mut - G | GATCACAGATTTTGGGCgGGCCAAACT GCTGGGTGCGGAAG (SEQ ID NO: 35) | TTTTGtGCtGGCCAA ACTGCTGG (SEQ ID NO: 13) | Transver sion sgRNA |
| PIK3CA p.E542K or c.1624G>A | WT - G | CACGAGATCCTCTCTCTgAAATCACTGA GCAGGAGAAAG (SEQ ID NO: 36) | TGCTCAGTGATTTcA GAGAGAGG (SEQ ID NO: 14) | Wild sgRNA |
| | Mut - A | CACGAGATCCTCTCTCTaAAATCACTGA GCAGGAGAAAG (SEQ ID NO: 37) | TGCTCAGTGATTTcA GAGAGAGG | |
| PIK3CA p.E545? or c.1633G>? | WT - G | CACGAGATCCTCTCTCTGAAATCACTgA GCAGGAGAAAG (SEQ ID NO: 38) | TCTCTCTGAAATCAC TgAGCAGG (SEQ ID NO: 15) | Wild sgRNA |
| | Mut - ?? (A or C or T) | CACGAGATCCTCTCTCTGAAATCACTaA GCAGGAGAAAG (SEQ ID NO: 39) | TCTCTCTGAAATCAC TgAGCAGG | |
| PIK3CA p.H1047? or c.3140A>? | WT - T | CATGAAACAAATGAATGATGCACaTCA TGGTGGCTGG (SEQ ID NO: 40) | CAAATGAATGATGC ACaTCATGG (SEQ ID NO: 16) | Wild sgRNA |
| | Mut - ?? (G or C or T) | CATGAAACAAATGAATGATGCACgTCA TGGTGGCTGG (SEQ ID NO: 41) | CAAATGAATGATGC ACaTCATGG | |
| BRAF p.V600E or c.1799T>A | WT - T | GATTTTGGTCTAGCTACAGtGAAATCTC GATGGAGTGGG (SEQ ID NO: 42) | TAGCTACAGtGAAAT CTCGATGG (SEQ ID NO: 17) | Wild sgRNA |
| | Mut - A | GATTTTGGTCTAGCTACAGaGAAATCTC GATGGAGTGGG (SEQ ID NO: 43) | TAGCTACAGtGAAAT CTCGATGG | |

[Table 1] shows guide RNAs for drug resistance mutations. All of the wild-type and mutant target sequences of selected genes for testing an *in vitro* transcription cleavage (IVT) analysis were subcloned into a T-blunt cloning vector for IVT testing, the positions of mutations in the wild-type target sequences are highlighted in bold, and the corresponding nucleotide mutations are shown in red (lower case). Mutational'?' marking means any nucleotide that is not a wild-type nucleotide. Small guide RNAs for each target were designed, and the PAM sequences are shown in blue (italic). Transversion mutations are shown in green. After confirming the IVT analysis, the samples were PCR amplified and analyzed through NGS.

### [Example 2]

### In vitro cleavage analysis using plasmids including wild-type and KRAS mutant (c.35G>C) sequences and sgRNA activity results

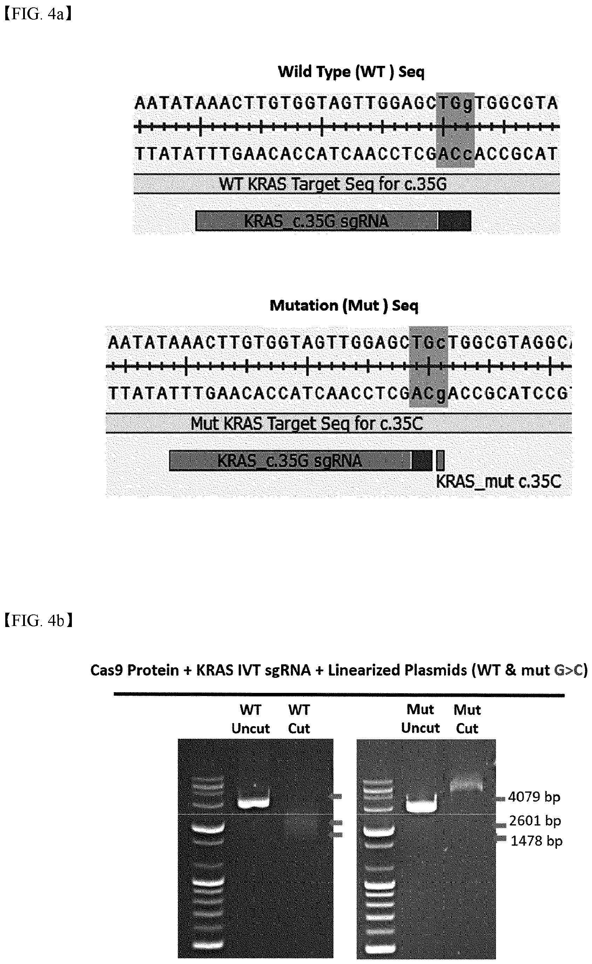

In order yo specifically cleave wild-type DNA, sgRNAs were designed to target 20 nucleotides together with the PAM site, but were disrupted by oncogenic mutations (c.35G>C) in the case of KRAS mutations (PAM). In order to test this approach by using an *in vitro* cleavage assay (IVT), target sequences were subcloned into T-blunt cloning vectors (wild-type and mutant KRAS sequences), and the plasmids were linearized with the vector's restriction enzymes. The linearized product was further cleaved by treatment with Cas9 nuclease and wild-type sequence specific sgRNA. After electrophoresis of wild-type or mutant DNA on a 0.8% gel, linearized fragments (not cleaved) and cleaved fragments (cleaved) were separated by size and indicated by red arrows (FIGS. 4a and 4b). DNA targeted in the wild type was completely cleaved by Cas9/sgRNA, but mutant target sequences were not cleaved by Cas9 and sgRNA. This is because mutations in the PAM site and these mutant alleles are not recognized by the Cas9 nuclease.

### [Example 3]

### In vitro cleavage assay using plasmids including wild-type or mutant sequences of oncogenic genes and sgRNA activity assays using Cas9 variants

In order to specifically cleave the wild-type target DNA, the sgRNA was designed to target a sequence of 20 nucleotides with the PAM site, but for EGFR mutations (c.2369C>T or p.T790M), the target mutation site is found within the trunk region of the sgRNA binding site. 1-base mismatch Cas9 cleavage activity within the trunk region (12-19) of the spacer of the sgRNA binding site is slightly or moderately reduced, but the cleavage activity is maintained. This activity of wild-type targeting sgRNA can also cleave mutant target alleles in cell-free DNA. In order to overcome this problem and avoid truncation of the mutant target allele, we generated a transversion mutation in the trunk region of the sgRNA (TmWT sgRNA). This TmWT-sgRNA is neither recognized nor cleaved by the Cas9 nuclease at the mutant allele because of its low resistance to both mutations. In order to test this approach by using an *in vitro* cleavage assay, target sequences were subcloned into T-blunt cloning vectors (wild-type and mutant EGFR sequences), and the plasmid was linearized with the vector's restriction enzymes. The linearized product was further cleaved by treatment with different combinations of Cas9 nuclease variants with wild-type sequence specific sgRNA (WT sgRNA) and inversion mutant sgRNA (TmWT sgRNA). After electrophoresis of wild-type or mutant DNA on a 0.8% gel, linearized fragments (uncleaved) and cleaved fragments (cleaved) were separated by size and indicated by red arrows. The DNA targeted in the wild type was completely cleaved by the Cas9 variant with WT-sgRNA, and even the mutant target sequence was completely cleaved by Cas9 and WT-sgRNA. However, the sgRNA (TmWT-sgRNA) in the transmutation mutant showed different cleavage patterns for Cas9 variants. This indicates that the TmWT-sgRNA is low resistant to both mutations and cannot completely cleave the mutation target allele.

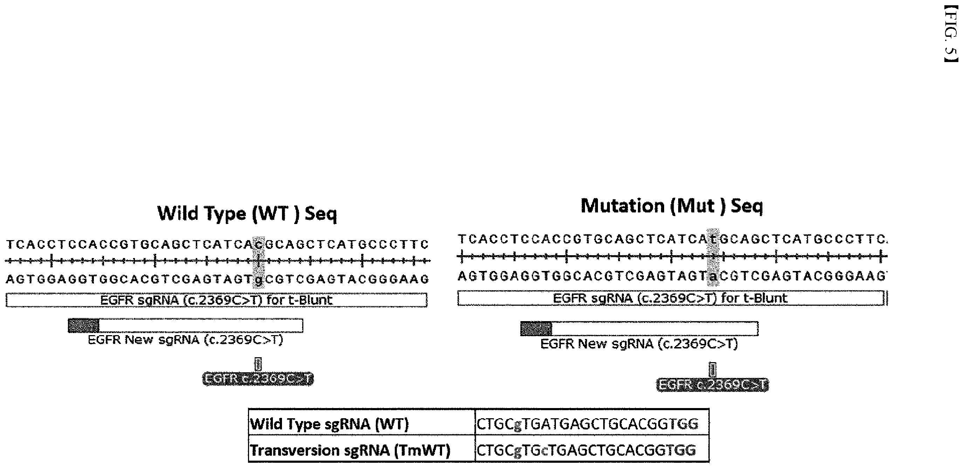

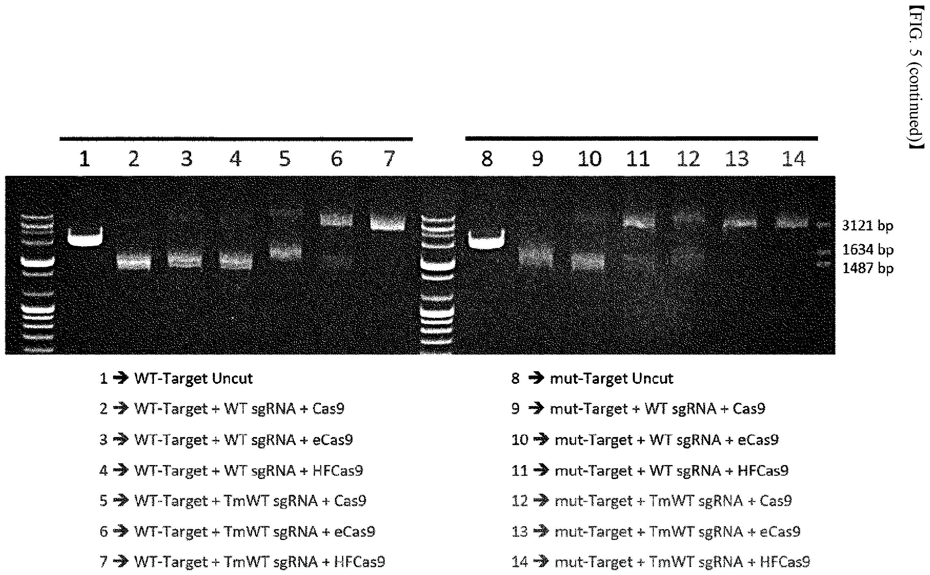

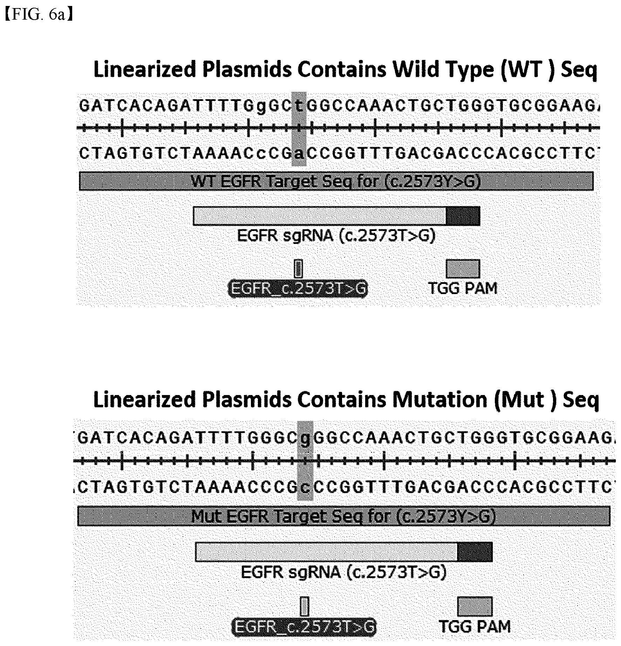

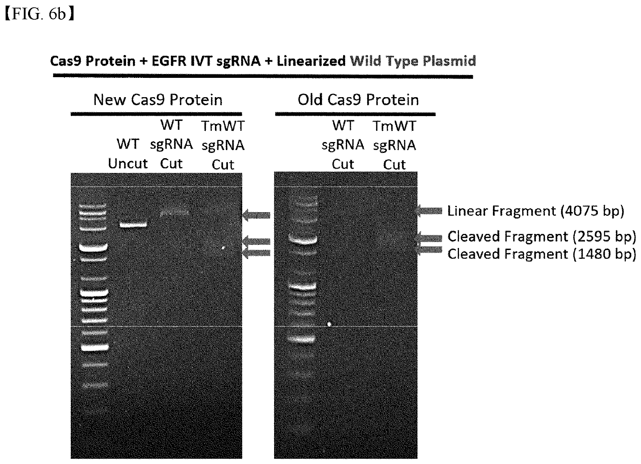

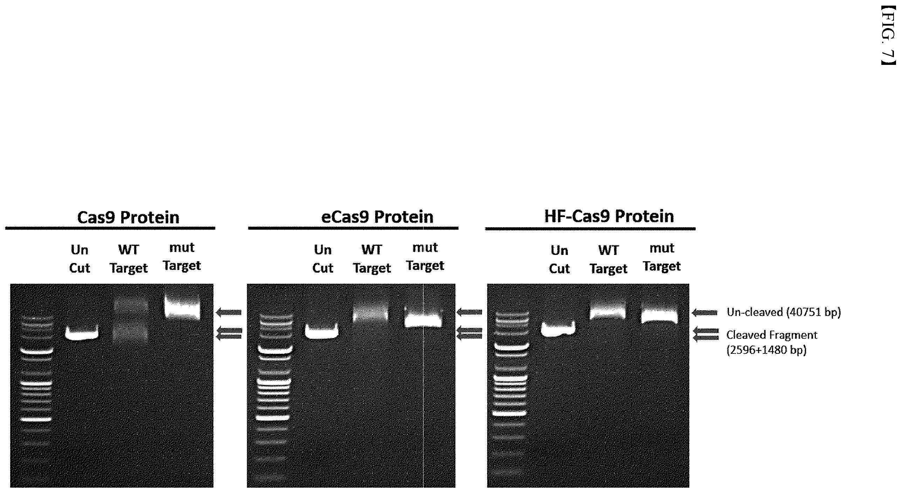

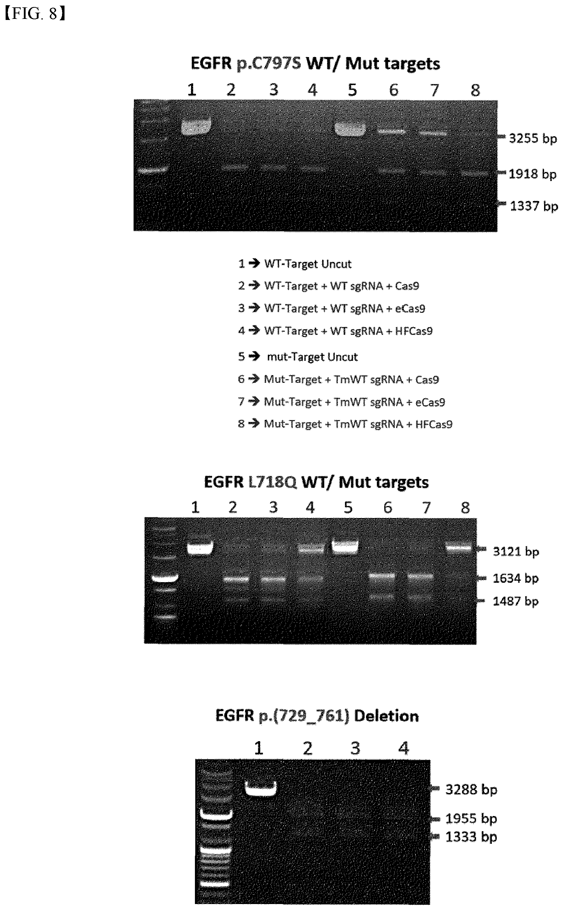

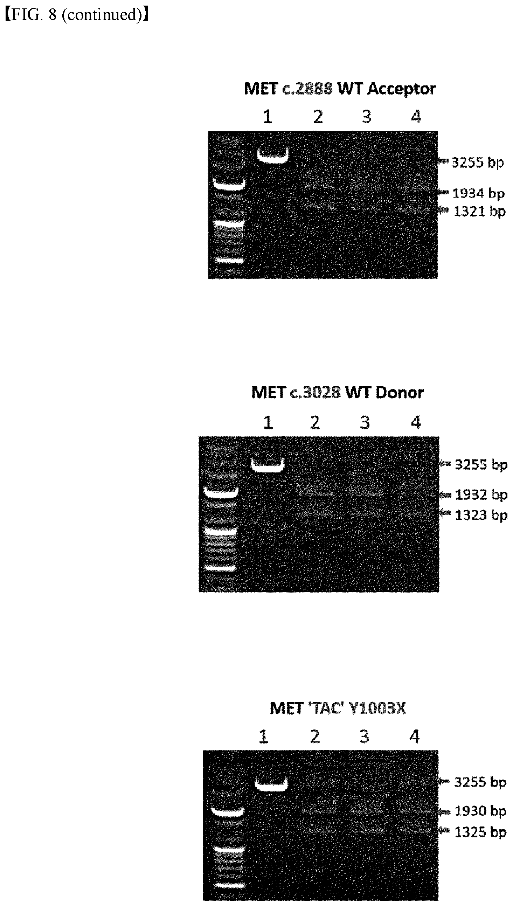

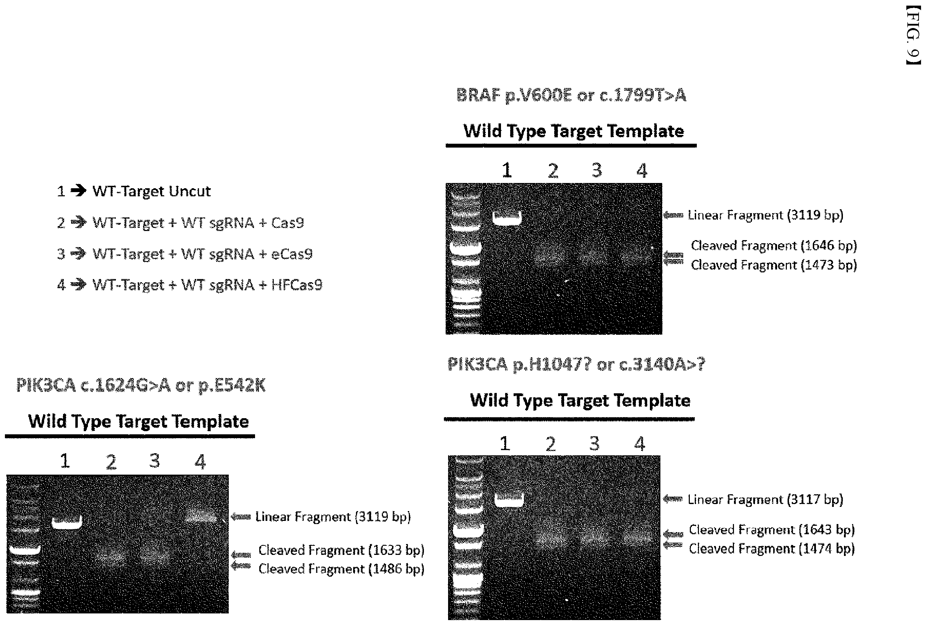

Based on the above strategy, the wild-type target allele was excised and removed to enrich the mutant allele (EGFR mutation (c.2369C>T or p.T790M; c.2573 T>G or p.L858R; p.C797S, L718Q or p.(729_761) deletion), MET mutation (c.2888, c.3028 or 'TAC'Y1003X), BRAF mutation (p.V600E or c.1799T>A) or PIK3CA mutation (c.1624G>A or p. E542K; p.H1047? or c.3140A>?)) (FIGS. 5 to 11). In the case of EGFR p.729-761 deletion and MET gene mutation, transversion mutant sgRNA was not designed because the mutation site was in the seed region.

### [Example 4]

### CUT-PCR-based enrichment analysis of EGFR mutants using wild-type and mutant plasmid mixtures at varying ratios

Analysis was performed by using various target sequences and guide RNAs in the same manner as in <Example 3> above. CUT-PCR experiments were performed on mixtures of plasmids at varying ratios including wild-type or mutant EGFR (c.2369C>T or p.T790M) sequences. DNA plasmids including the wild-type and mutant sequences were mixed at various ratios, and Cas9 mutant digestion was performed *in vitro.* The plasmid mixture was treated with the transversion mutant sgRNA (TmWT-sgRNA) together with the Cas9 variant to cleave wild-type DNA. After treatment, samples were purified by using a PCR cleanup kit. In order to quantify target specific cleavage, the fold increase of the target sequence was compared to the fold increase of the untreated control product. The amount of EGFR target region with respect to the untreated control product in each lane was calculated by NGS analysis. For the EGFR (c.2369C>T or p.T790M) mutation, the plasmid mixture in the presence of the mutant plasmid was subjected to targeted deep sequencing after treating CUT-PCR (orange or pink bars) or without treatment (purple or blue bars). It was originally mixed with the wild-type plasmid at a ratio of 0.5 to 5%. The resulting PCR amplicons were subjected to paired-end sequencing with MiSeq. NGS results were analyzed by comparing wild-type and mutant sequence fold changes for all mixing ratios of mutant DNA fragments in both of Cas9 and eCas9 varients with TmWT-sgRNA.

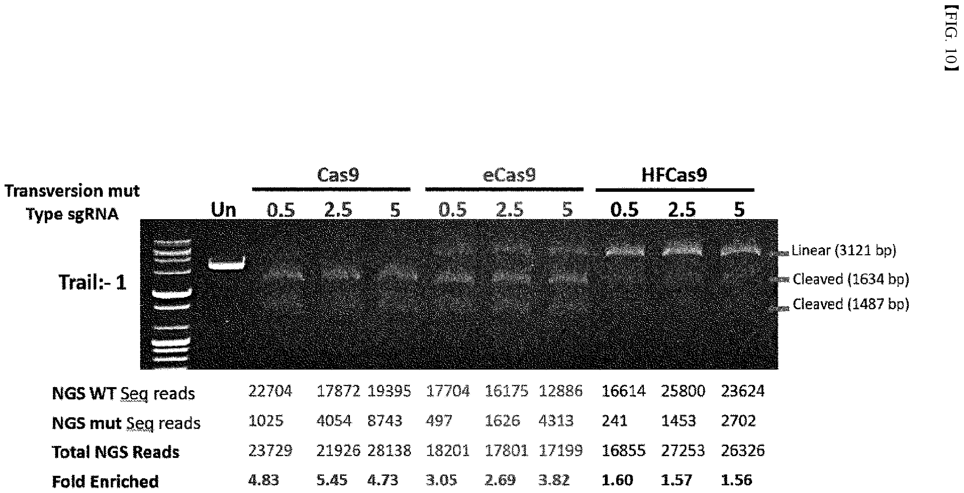

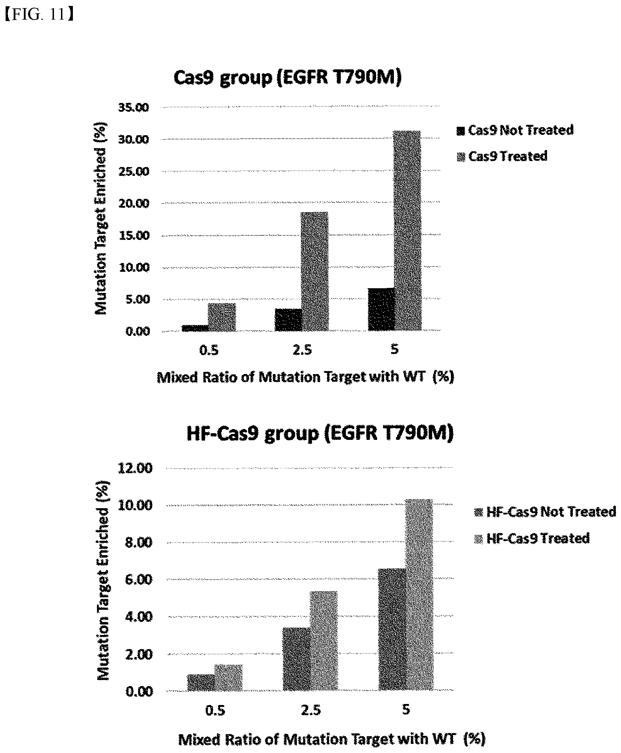

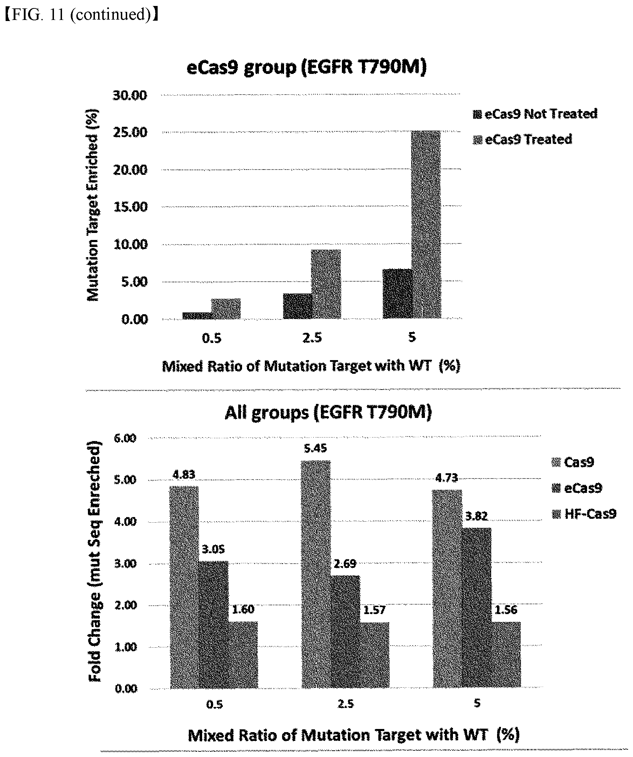

Mutant sequences were abundant by 4.8 to 5.4 folds (FIGS. 10 and 11).

### [Example 5]

### CUT-PCR-based enrichment analysis of multiple gene mutations using wild-type and mutant plasmid mixtures at varying ratios

Analysis was performed by using various target sequences and guide RNAs in the same manner as in <Example 3> above. CUT-PCR experiments for plasmid mixtures at various rations including the wild-type or mutant sequences of KRAS (c.35 G>C) and EGFR (c.2369 C>T, c.2390 G>C, and c.2153 T>A) were performed. DNA plasmids including the wild-type and mutant sequences of multiple gene mutations were mixed at various ratios, and Cas9 variant cleavage was performed *in vitro.* The plasmid mixture was treated with a mixed sgRNA corresponding to each gene with a Cas9 variant to cleave the wild-type DNA. After treatment, samples were purified by using a PCR cleanup kit. In order to quantify multi-target specific cleavage, the fold increase of the target sequence was compared to the fold increase of the untreated control product. The amount of each target area with respect to the untreated control product in each lane was calculated by NGS analysis. NGS results were analyzed by comparing the percentages of the wild-type and mutant sequences for all mixing ratios of mutant DNA fragments in both of Cas9 and eCas9 variants to multiple sgRNAs.

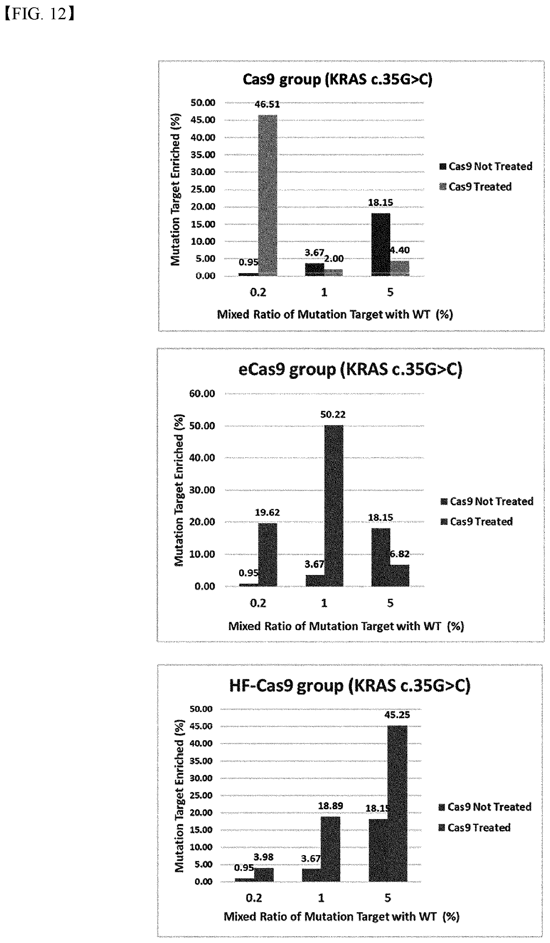

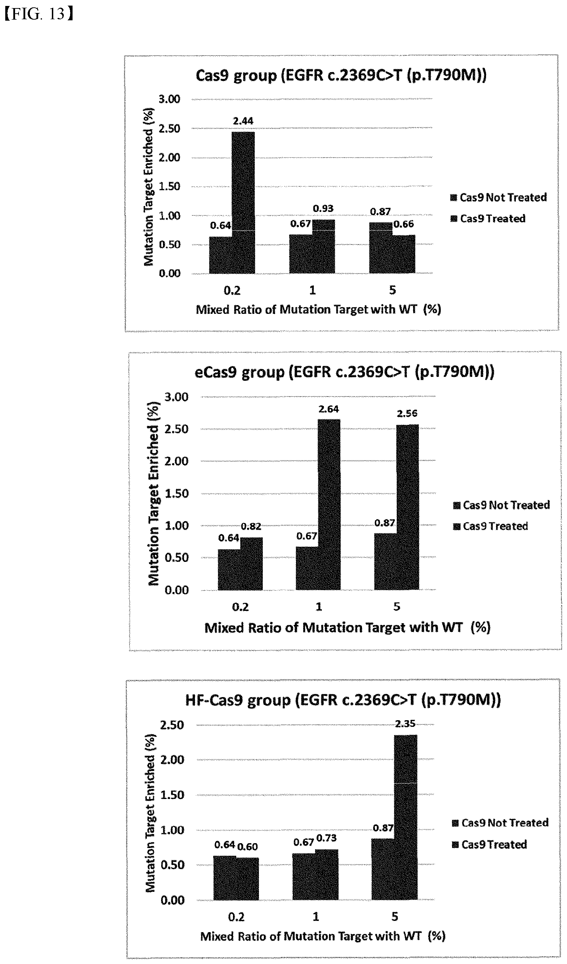

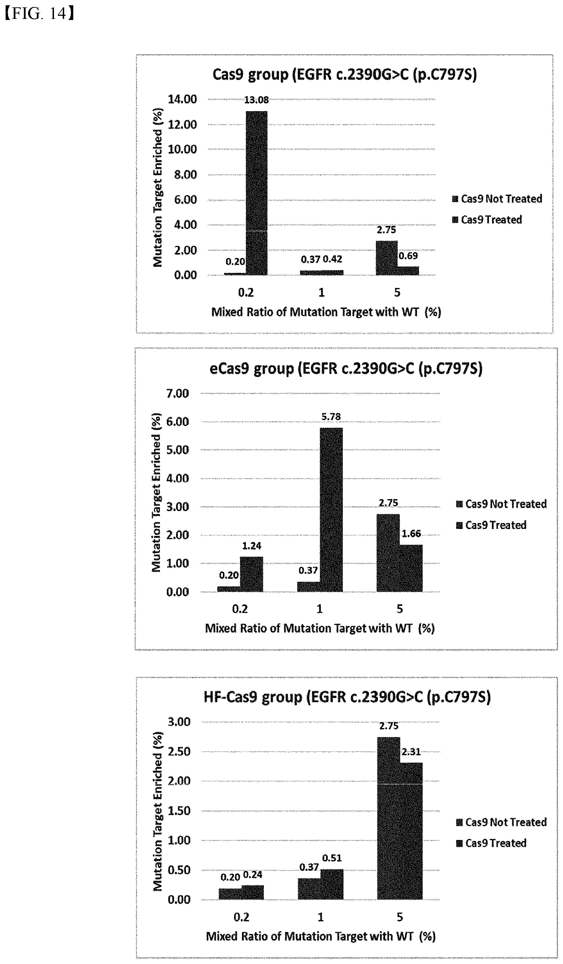

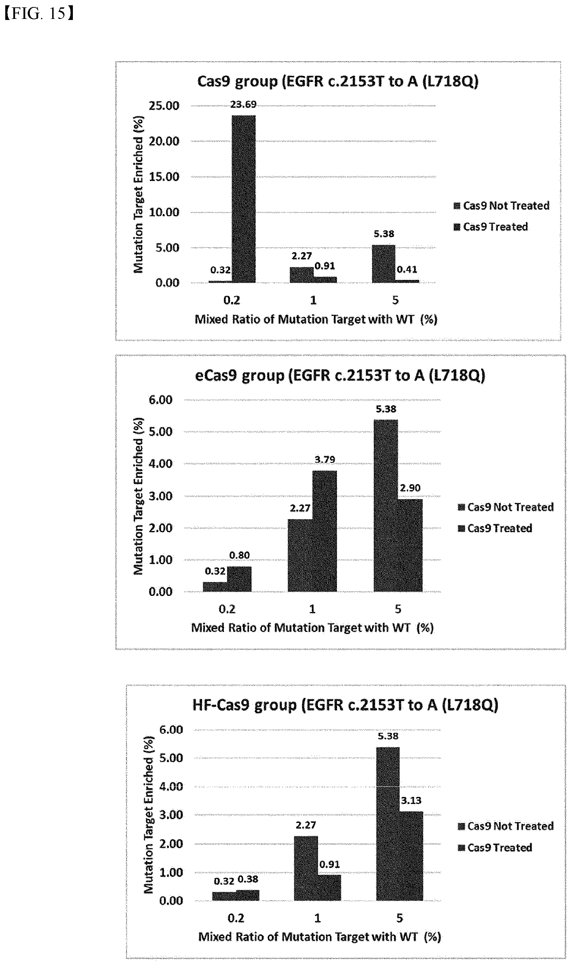

Mutant sequences were abundant in the range of 0.6 to 46.5% (FIGS. 12 to 15).

### [Example 6]

### CUT-PCR-based enrichment of multiple mutations using cfDNA reference standard set

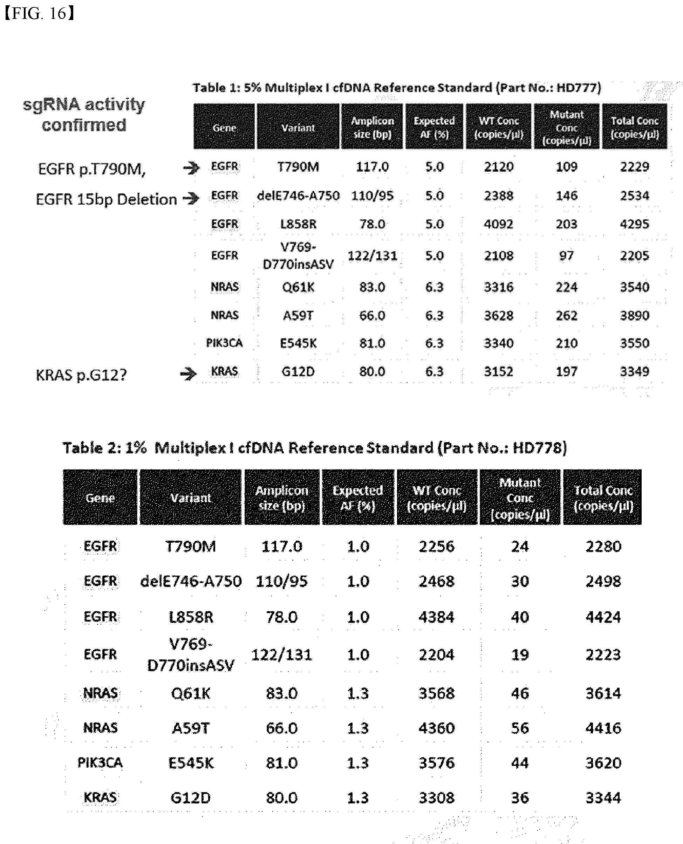

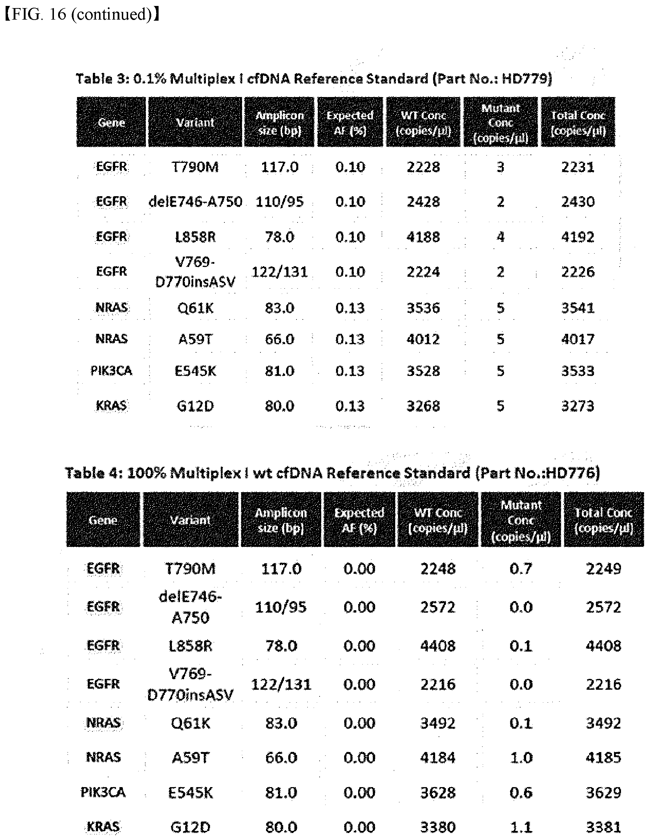

In order to confirm or demonstrate sgRNA activity and experimental methods for the enrichment of mutant alleles in cfDNA, commercially available multiple cfDNA samples were utilized as reference standards, and the activities of 3 sgRNAs were confirmed. The multiplex cfDNA standard sample included 4 genes with 8 different mutations and included 3 proportions of each gene (FIG. 16).

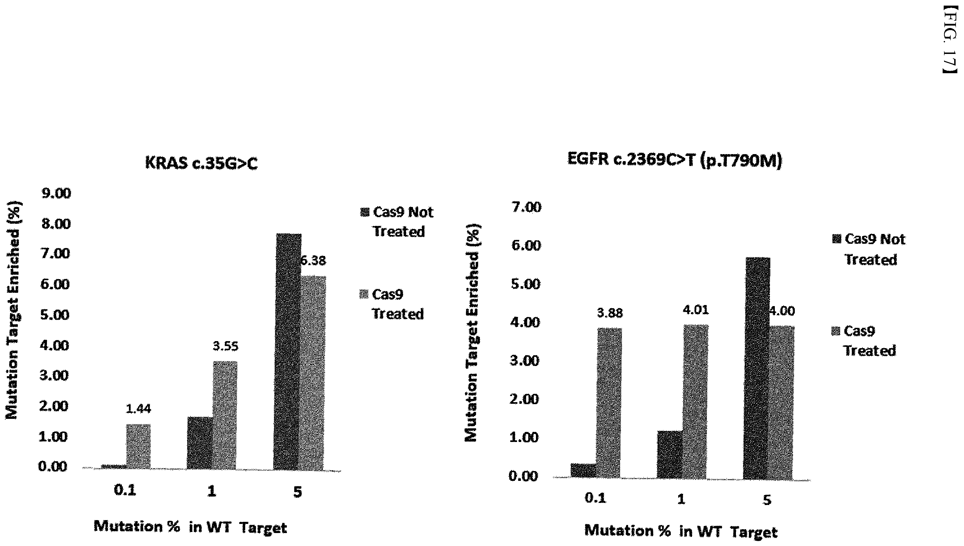

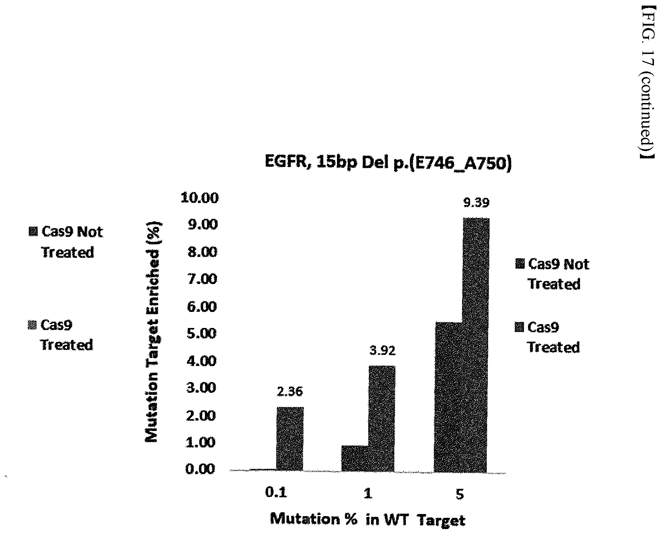

Experimental methods to enrich for the mutant alleles in sgRNA activity and cfDNA were confirmed or demonstrated. The reference standard mixture was treated with the sgRNA corresponding to each gene with SpCas9 to cleave the wild-type DNA. After treatment, samples were purified by using a PCR cleanup kit. In order to quantify multi-target specific cleavage, the percentage of each target sequence was compared to the percentage of the untreated control product. The amount of each target area with respect to the untreated control product in each lane was calculated by NGS analysis. NGS results were analyzed by comparing untreated and treated sample ratios for all mixed ratios of cfDNA at different ratios from SpCas9 and multi-sgRNA, and mutant sequences were abundant in the range of 1.44 to 9.39% (FIG. 17).

In addition, the mixed sgRNA was treated with KRAS, EGFR-T790M or EGFR 729-761bp deletion samples, and then, the results of performing an in *vitro* cleavage assay are shown in [Table 2].

**[Table 2]**

| Cas9 with Mixed-sgRNA's Treated for Multiplex 1 | KRAS PCR | | | | EGFR T790M PCR | | | | EGFR Deletion PCR | | | |
|---|---|---|---|---|---|---|---|---|---|---|---|---|
| | 0.1 | 1 | 5 | 100 | 0.1 | 1 | 5 | 100 | 0.1 | 1 | 5 | 100 |
| Cas9 Not Treated | 0.10 | 1.71 | 7.77 | 0.05 | 0.34 | 1.24 | 5.79 | 0.17 | 0.04 | 0.94 | 5.53 | 0.05 |
| Cas9 Treated | 2.33 | 9.91 | 4.12 | 0.09 | 0.05 | 0.02 | 7.92 | 0.05 | 4.59 | 0.02 | 25.96 | 0.27 |
| Fold Change(mut Seq Enreached) | 22.28 | 5.81 | 0.53 | 1.87 | 0.14 | 0.02 | 1.37 | 0.32 | 102.34 | 0.02 | 4.69 | 5.86 |

### [Example 7]

### Ultra-sensitive detection of mutational targets in cell-free DNA from patient samples

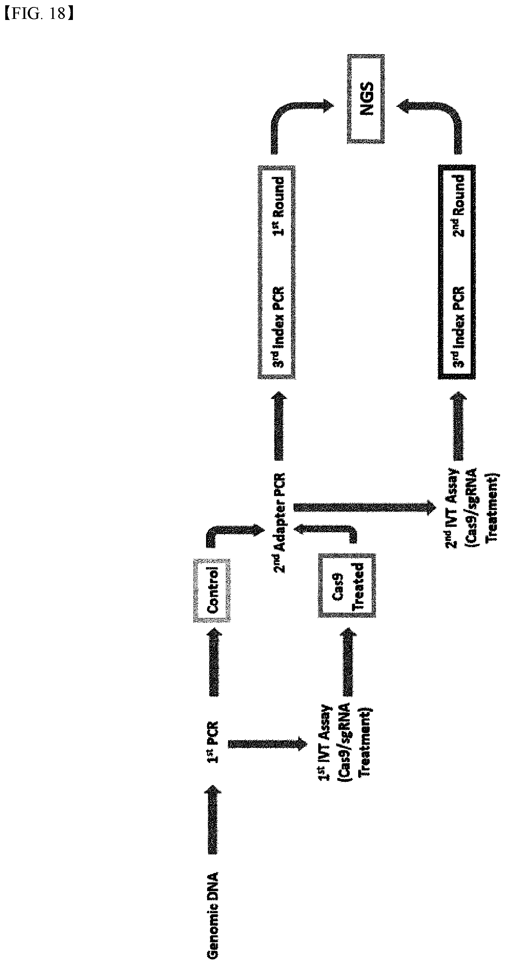

Mutation targets in cell-free DNA were detected by using the guide RNA of the present invention in the blood of lung cancer patients with mutations (FIG. 18).

Table 3 below shows patient samples with the L858R mutation, and Table 4 shows patient samples with the T790M and E19del mutations.

**[Table 3]**

| ID | Date | Gender | Classification |
|---|---|---|---|
| LA-29 | 2015-06-19 | Male | Terminal stage |
| LA-209 | 2016-10-04 | Female | Late stage |
| LA-292 | 2019-04-19 | Male | Late stage |

**[Table 4]**

| ID | Date | Gender | Classification |
|---|---|---|---|
| LA-232 | 2017-02-14 | Male | Late stage |
| LA-322 | 2019-10-07 | Male | Late stage |
| LA-323 | 2019-10-14 | Female | Late stage |

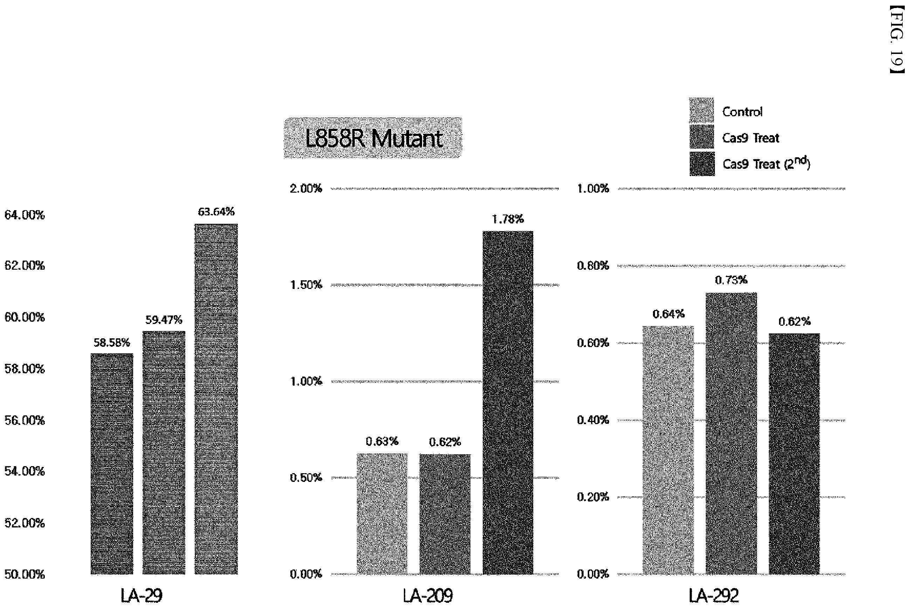

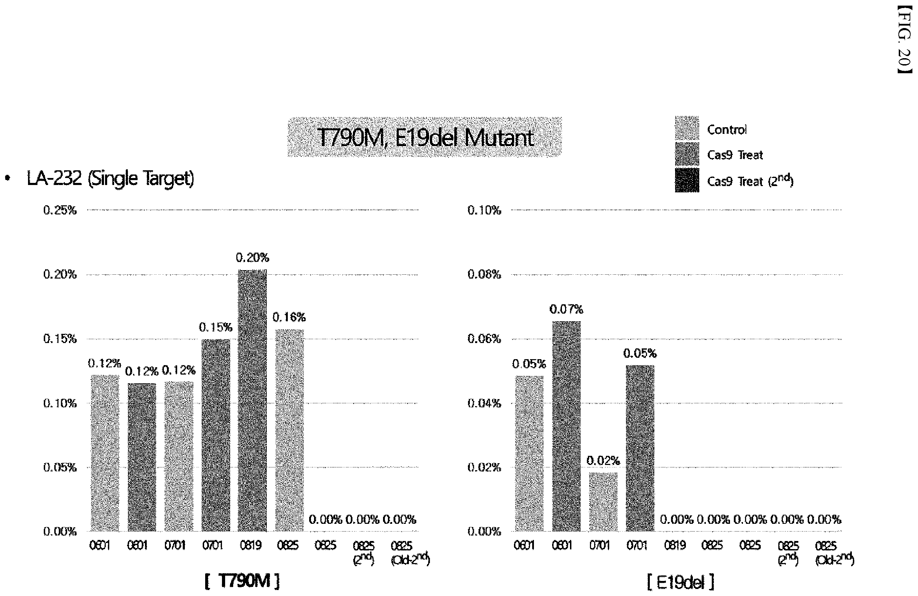

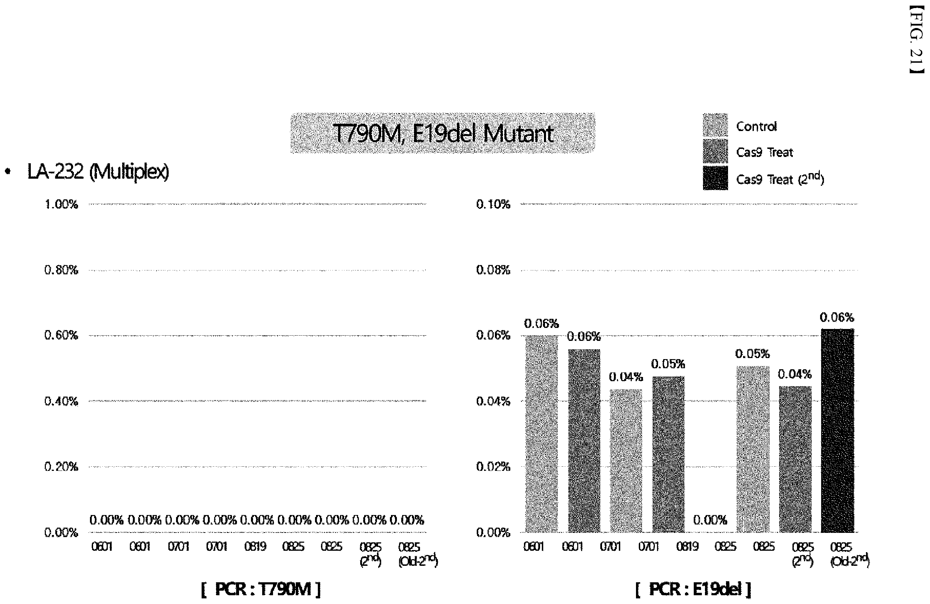

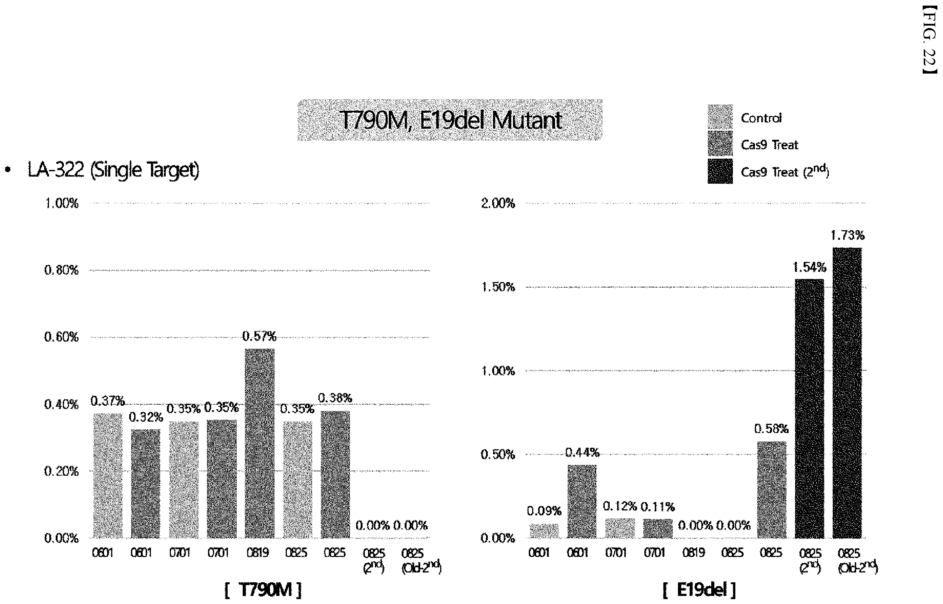

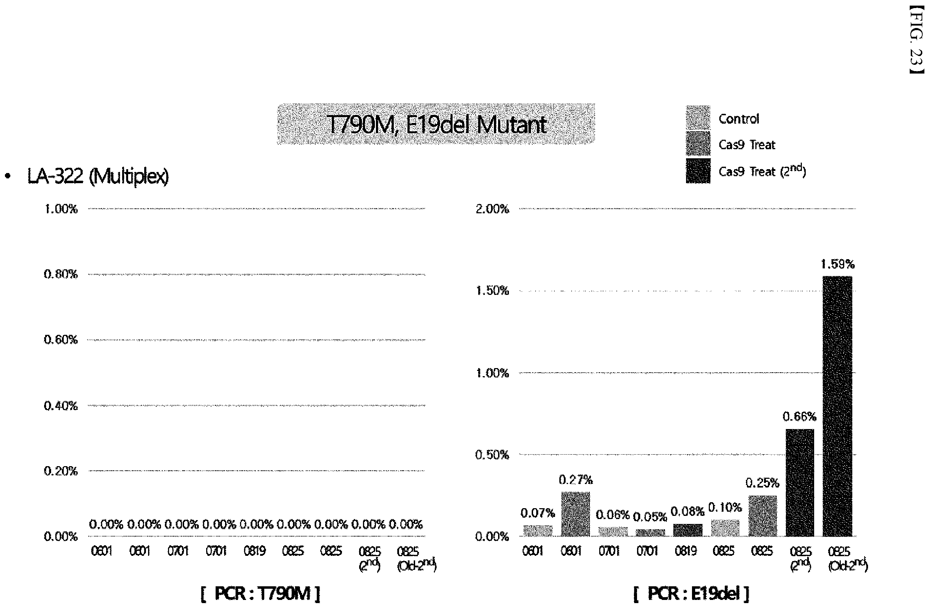

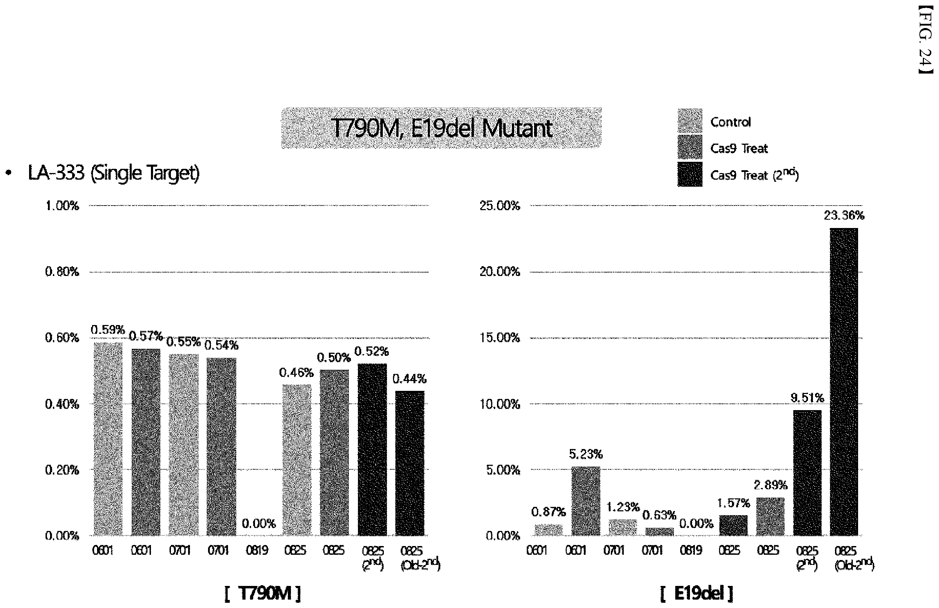

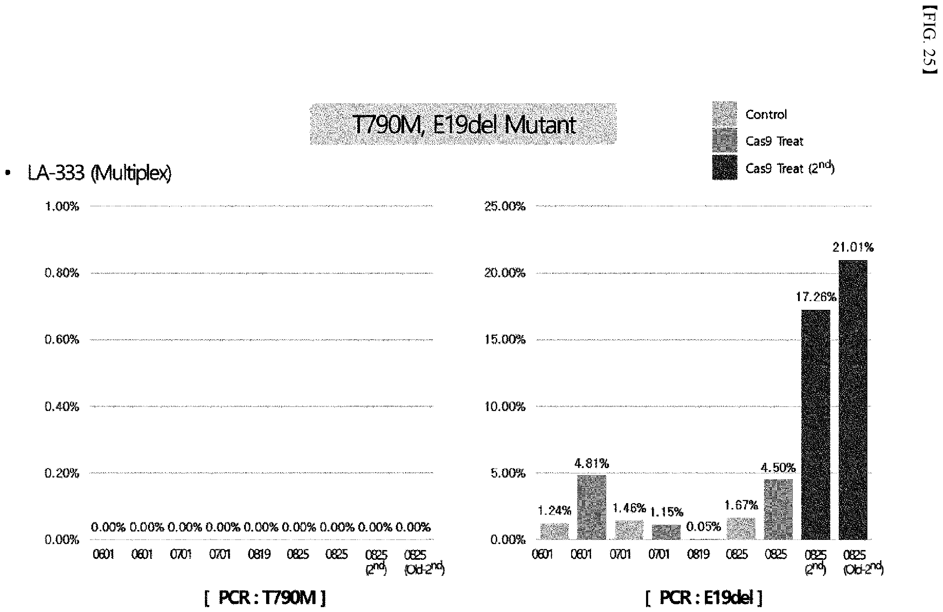

Analysis was performed by using various target sequences and guide RNAs in the same manner as in <Example 3>. The NGS results were analyzed by comparing the proportion of untreated samples for each patient's cfDNA with SpCas9 and the corresponding sgRNA, and mutant sequences were detected in abundance (FIGS. 19 to 25).

### [Industrial Applicability]

When detecting cancer mutations with the CRISPR/Cas9 system using the guide RNA of the present invention, only wild-type cell-free DNA (cfDNA) is cleaved and oncogenic mutant DNAis not cleaved, and thus, compared to when the guide RNA of the existing CRISPR/Cas9 system is used, it can be effectively used to selectively amplify only oncogenic mutant DNA. In particular, when the guide RNA (wild-type guide RNA) of the present invention and the transversion guide RNA (transversion sgRNA) are used together, the effects of cleaving only cfDNA and selectively amplifying only oncogenic mutant DNA can be significantly increased, and thus, it has industrial applicability.

### [Sequence List Free Text]

SEQ ID NO: 1 is a wild-type guide RNA (wild sgRNA) which is capable of detecting EGFR (p.T790M or c.2369C>T) mutation, and when it is used in an *in vitro* cleavage analysis, it may have SEQ ID NO: 18 as a target sequence.

SEQ ID NO: 2 is a transversion guide RNA (transversion sgRNA) which is capable of detecting EGFR (p.T790M or c.2390 G>C) mutation, and when it is used in an *in vitro* cleavage analysis, it may have SEQ ID NO: 19 as a target sequence. When using a guide RNA including the nucleotide sequence of SEQ ID NO: 2, the mutant sequence may be amplified by 1% to 32%.

SEQ ID NO: 3 is a wild-type guide RNA (wild sgRNA) which is capable of detecting EGFR (p.C797S) mutation, and when it is used in an *in vitro* cleavage analysis, it may have SEQ ID NO: 20 as a target sequence.

SEQ ID NO: 4 is a transversion guide RNA (transversion sgRNA) which is capable of detecting EGFR (p.C797S) mutation, and when it is used in an *in vitro* cleavage analysis, it may have SEQ ID NO: 21 as a target sequence. When using a guide RNA including the nucleotide sequence of SEQ ID NO: 4, the mutant sequence may be amplified by 1% to 13%.

SEQ ID NO: 5 is a wild-type guide RNA (wild sgRNA) which is capable of detecting MET c.2888 Int13-Exon14 Acceptor (Exon-14 Skipping) mutation, and when it is used in an *in vitro* cleavage analysis, it may have SEQ ID NO: 22 or 23 as a target sequence.

SEQ ID NO: 6 is a wild-type guide RNA (wild sgRNA) which is capable of detecting MET c.3028 Exon14-Int14 Donor (Exon-14 Skipping) mutation, and when it is used in an *in vitro* cleavage analysis, it may have SEQ ID NO: 24 or 25 as a target sequence.

SEQ ID NO: 7 is a wild-type guide RNA (wild sgRNA) which is capable of detecting MET 'TAC' Y1003X (Exon-14 Skipping) mutation, and when it is used in an *in vitro* cleavage analysis, it may have SEQ ID NO: 26 or 27 as a target sequence.

SEQ ID NO: 8 is a wild-type guide RNA (wild sgRNA) which is capable of detecting EGFR (L718Q or c.2153 T> A) mutation, and when it is used in an *in vitro* cleavage analysis, it may have SEQ ID NO: 28 as a target sequence.

SEQ ID NO: 9 is a transversion guide RNA (transversion sgRNA) which is capable of detecting EGFR (L718Q) mutation, and when it is used in an *in vitro* cleavage analysis, it may have SEQ ID NO: 29 as a target sequence. When using a guide RNA including the nucleotide sequence of SEQ ID NO: 9, the mutant sequence may be amplified by 0.8% to 23.7%.

SEQ ID NO: 10 is a wild-type guide RNA (wild sgRNA) which is capable of detecting KRAS (c.35G) mutation, and when it is used in an *in vitro* cleavage analysis, it may have SEQ ID NO: 30 or 31 as a target sequence. When using a guide RNA including the nucleotide sequence of SEQ ID NO: 10, the mutant sequence may be amplified by 1.5% to 50%.

SEQ ID NO: 11 is a wild-type guide RNA (wild sgRNA) which is capable of detecting EGFR, 15bp Del p. (729_761) mutation, and when it is used in an *in vitro* cleavage analysis, it may have SEQ ID NO: 32 or 33 as a target sequence. When using a guide RNA including the nucleotide sequence of SEQ ID NO: 11, the mutant sequence may be amplified by 0.5% to 23%.

SEQ ID NO: 12 is a wild-type guide RNA (wild sgRNA) which is capable of detecting EGFR (p.L858R or c.2573 T>G) mutation, and when it is used in an *in vitro* cleavage analysis, it may have SEQ ID NO: 34 as a target sequence.

SEQ ID NO: 13 is a transversion guide RNA (transversion sgRNA) which is capable of detecting EGFR (p.L858R) mutation, and when it is used in an *in vitro* cleavage analysis, it may have SEQ ID NO: 35 as a target sequence. When using a guide RNA including the nucleotide sequence of SEQ ID NO: 13, the mutant sequence may be amplified by 1.7% to 63%.

SEQ ID NO: 14 is a wild-type guide RNA (wild sgRNA) which is capable of detecting PIK3CA (p.E542K or c.1624G>A) mutation, and when it is used in an *in vitro* cleavage analysis, it may have SEQ ID NO: 36 or 37 as a target sequence.

SEQ ID NO: 15 is a wild-type guide RNA (wild sgRNA) which is capable of detecting PIK3CA (p.E545? or c.1633G>?) mutation, and when it is used in an *in vitro* cleavage analysis, it may have SEQ ID NO: 38 or 39 as a target sequence.

SEQ ID NO: 16 is a wild-type guide RNA (wild sgRNA) which is capable of detecting PIK3CA (p.H1047? or c.3140A>?) mutation, and when it is used in an *in vitro* cleavage analysis, it may have SEQ ID NO: 40 or 41 as a target sequence.

SEQ ID NO: 17 is a wild-type guide RNA (wild sgRNA) which is capable of detecting PIK3CA (p.V600E or c.1799T>A) mutation, and when it is used in an *in vitro* cleavage analysis, it may have SEQ ID NO: 42 or 43 as a target sequence.

SEQ ID NO: 18 represents a target sequence of the wild-type guide RNA (wild sgRNA) of SEQ ID NO: 1 which is capable of detecting EGFR (p.T790M or c.2369C>T) mutation.

SEQ ID NO: 19 represents a target sequence of the transversion guide RNA (transversion sgRNA) of SEQ ID NO: 2 which is capable of detecting EGFR (p.T790M or c.2390 G>C) mutation.

SEQ ID NO: 20 represents a target sequence of the wild-type guide RNA (wild sgRNA) of SEQ ID NO: 3 which is capable of detecting EGFR (p.C797S) mutation.

SEQ ID NO: 21 represents a target sequence of the conversion mutation guide RNA (transversion sgRNA) of SEQ ID NO: 4 which is capable of detecting EGFR (p.C797S) mutation.

SEQ ID NO: 22 represents a target sequence of the wild-type guide RNA (wild sgRNA) of SEQ ID NO: 5 which is capable of detecting MET c.2888 Int13-Exon14 Acceptor (Exon-14 Skipping) mutation.

SEQ ID NO: 23 represents a target sequence of the wild-type guide RNA (wild sgRNA) of SEQ ID NO: 5 which is capable of detecting MET c.2888 Int13-Exon14 Acceptor (Exon-14 Skipping) mutation.

SEQ ID NO: 24 represents a target sequence of the wild-type guide RNA (wild sgRNA) of SEQ ID NO: 6 which is capable of detecting MET c.3028 Exon14-Int14 Donor (Exon-14 Skipping) mutation.

SEQ ID NO: 25 represents a target sequence of the wild-type guide RNA (wild sgRNA) of SEQ ID NO: 6 which is capable of detecting MET c.3028 Exon14-Int14 Donor (Exon-14 Skipping) mutation.

SEQ ID NO: 26 represents a target sequence of the wild-type guide RNA (wild sgRNA) of SEQ ID NO: 7 which is capable of detecting MET 'TAC' Y1003X (Exon-14 Skipping) mutation.

SEQ ID NO: 27 represents a target sequence of the wild-type guide RNA (wild sgRNA) of SEQ ID NO: 7 which is capable of detecting MET 'TAC' Y1003X (Exon-14 Skipping) mutation.

SEQ ID NO: 28 represents a target sequence of the wild-type guide RNA (wild sgRNA) of SEQ ID NO: 8 which is capable of detecting EGFR (L718Q or c.2153 T> A) mutation.

SEQ ID NO: 29 represents a target sequence of the conversion mutation guide RNA (transversion sgRNA) of SEQ ID NO: 9 which is capable of detecting EGFR (L718Q) mutation.

SEQ ID NO: 30 represents a target sequence of the wild-type guide RNA (wild sgRNA) of SEQ ID NO: 10 which is capable of detecting KRAS (c.35G) mutation.

SEQ ID NO: 31 represents a target sequence of the wild-type guide RNA (wild sgRNA) of SEQ ID NO: 10 which is capable of detecting KRAS (c.35G) mutation.

SEQ ID NO: 32 represents a target sequence of the wild-type guide RNA (wild sgRNA) of SEQ ID NO: 11 which is capable of detecting EGFR, 15 bp Del p. (729_761) mutation.

SEQ ID NO: 33 represents a target sequence of the wild-type guide RNA (wild sgRNA) of SEQ ID NO: 11 which is capable of detecting EGFR, 15 bp Del p. (729_761) mutation.

SEQ ID NO: 34 represents a target sequence of the wild-type guide RNA (wild sgRNA) of SEQ ID NO: 12 which is capable of detecting EGFR (p.L858R or c.2573 T>G) mutation.

SEQ ID NO: 35 represents a target sequence of the transversion guide RNA (transversion sgRNA) of SEQ ID NO: 13 which is capable of detecting EGFR (p.L858R) mutation.

SEQ ID NO: 36 represents a target sequence of the wild-type guide RNA (wild sgRNA) of SEQ ID NO: 14 which is capable of detecting PIK3CA (p.E542K or c.1624G>A) mutation.

SEQ ID NO: 37 represents a target sequence of the wild-type guide RNA (wild sgRNA) of SEQ ID NO: 14 which is capable of detecting PIK3CA (p.E542K or c.1624G>A) mutation.

SEQ ID NO: 38 represents a target sequence of the wild-type guide RNA (wild sgRNA) of SEQ ID NO: 15 which is capable of detecting PIK3CA (p.E545? or c.1633G>?) mutation.

SEQ ID NO: 39 represents a target sequence of the wild-type guide RNA (wild sgRNA) of SEQ ID NO: 15 which is capable of detecting PIK3CA (p.E545? or c.1633G>?) mutation.

SEQ ID NO: 40 represents a target sequence of the wild-type guide RNA (wild sgRNA) of SEQ ID NO: 16 which is capable of detecting PIK3CA (p.H1047? or c.3140A>?) mutation.

SEQ ID NO: 41 represents a target sequence of the wild-type guide RNA (wild sgRNA) of SEQ ID NO: 16 which is capable of detecting PIK3CA (p.H1047? or c.3140A>?) mutation.

SEQ ID NO: 42 represents a target sequence of the wild-type guide RNA (wild sgRNA) of SEQ ID NO: 17 which is capable of detecting PIK3CA (p.V600E or c.1799T>A) mutation.

SEQ ID NO: 43 represents a target sequence of the wild-type guide RNA (wild sgRNA) of SEQ ID NO: 17 which is capable of detecting PIK3CA (p.V600E or c.1799T>A) mutation.

## Claims

1. A guide RNA, which is a guide RNA used in the CRISPR/Cas9 system, wherein the guide RNA cleaves wild-type cell-free DNA.

2. The guide RNA of claim 1, wherein the guide RNA comprises at least any one nucleotide sequence selected from the group consisting of SEQ ID NOs: 1 to 17.

3. The guide RNA of claim 1, wherein in the guide RNA, a transversion mutation has occurred in at least any one region selected from the group consisting of the trunk region, semi-seed region and seed region of the guide RNA.

4. The guide RNA of claim 3, wherein the guide RNA comprises at least any one nucleotide sequence selected from the group consisting of SEQ ID NO: 2, SEQ ID NO: 4, SEQ ID NO: 9 and SEQ ID NO: 13.

5. The guide RNA of claim 1, wherein the guide RNA is capable of detecting at least any one cancer-related mutation selected from the group consisting of EGFR, MET, KRAS, PIK3CA and BRAF.

6. The guide RNA of claim 5, wherein when detecting the EGFR mutation, the guide RNA comprises at least any one nucleotide sequence selected from the group consisting of SEQ ID NOs: 1 to 4, 8, 9 and 11 to 13.

7. The guide RNA of claim 5, wherein when detecting the MET mutation, the guide RNA comprises at least any one nucleotide sequence selected from the group consisting of SEQ ID NOs: 5 to 7.

8. The guide RNA of claim 5, wherein when detecting the KRAS mutation, the guide RNA comprises the nucleotide sequence of SEQ ID NO: 10.

9. The guide RNA of claim 5, wherein when detecting the PIK3CA mutation, the guide RNA comprises at least any one nucleotide sequence selected from the group consisting of SEQ ID NOs: 14 to 16.

10. The guide RNA of claim 5, wherein when detecting the BRAF mutation, the guide RNA comprises the nucleotide sequence of SEQ ID NO: 17.

11. A composition for diagnosing cancer or predicting cancer prognosis, comprising a guide RNA of CRISPR/Cas9 comprising at least any one nucleotide sequence selected from the group consisting of SEQ ID NOs: 1 to 17.

12. The composition of claim 11, wherein in the guide RNA, a transversion mutation has occurred in at least any one region selected from the group consisting of the trunk region, semi-seed region and seed region of the guide RNA.

13. The composition of claim 11, wherein the guide RNA is capable of detecting at least any one cancer-related mutation selected from the group consisting of EGFR, MET, KRAS, PIK3CA and BRAF.

14. The composition of claim 11, wherein the guide RNA cleaves wild-type cell-free DNA.

15. The composition of claim 11, wherein the cancer is at least any one cancer selected from the group consisting of lung cancer, pancreatic cancer, colon cancer, stomach cancer, breast cancer, kidney cancer, liver cancer, blood cancer, bladder cancer and uterine cancer.

16. A kit for diagnosing cancer or predicting cancer prognosis, comprising the composition of claim 11.

17. A method for providing information for diagnosing cancer or predicting cancer prognosis, comprising the step of treating the composition of claim 11 to a sample of a patient.
